# EUROPEAN PATENT SPECIFICATION

(11) **EP 3 218 351 B1**
(45) Date of publication and mention of the grant of the patent: **19.06.2019**
(21) Application number: 15808087.9
(22) Date of filing: 11.11.2015
(51) Int. Cl.: C07C 211/06, C07C 211/07, C07C 211/17, C07C 233/47, C07C 211/35, A61K 31/216

(54) **A METHOD FOR THE PREPARATION, ISOLATION AND PURIFICATION OF PHARMACEUTICALLY APPLICABLE FORMS OF AHU-377**
VERFAHREN ZUR HERSTELLUNG, ISOLIERUNG UND REINIGUNG VON PHARMAZEUTISCH ANWENDBAREN FORMEN VON AHU-377
PROCÉDÉ DE PRÉPARATION, D'ISOLATION ET DE PURIFICATION DE FORMES PHARMACEUTIQUEMENT APPLICABLES DE AHU-377

(30) Priority: 14.11.2014 CZ 20140792; 27.10.2015 CZ 20150758
(43) Date of publication of application: 20.09.2017
(73) Proprietor: Zentiva K.S., 102 37 Praha 10 (CZ)
(72) Inventor: HALAMA, Ales, 530 09 Pardubice (CZ); ZVATORA, Pavel, 798 03 Plumlov (CZ); DAMMER, Ondrej, 253 01 Hostivice (CZ); STACH, Jan, 190 16 Praha 9 (CZ); ZAPADLO, Michal, CZ-549 41 Cerveny Kostelec (CZ); KREJCIK, Lukas, 190 17 Praha 9 (CZ); VOSLAR, Michal, 103 00 Praha 10 (CZ)
(74) Representative: Jirotkova, Ivana
(86) International application number: PCT/CZ2015/000136
(87) International publication number: WO 2016/074651

(56) References cited:
- EP-A1- 0 555 175
- WO-A1-03/059345
- WO-A1-2007/056546
- WO-A1-2008/031567
- WO-A2-2008/083967
- CN-A- 105 168 205
- "Definition of Alkyl", 12 October 2018 (2018-10-12), Wikipedia

## Description

### Technical Field

The compound AHU-377, chemically (2*R*,4*S*)-5-(biphenyl-4-yl)-4-[(3-carboxypropionyl)-amino]-2-methylpentanoic acid ethyl ester of formula (**1**), also known as sacubitril, is a constituent of a drug for the treatment of hypertension and heart failure. It is especially the crystalline supramolecular complex of the sodium salt of AHU-377 and the disodium salt of valsartan of formula (2) in the 1:1 ratio of the components, which crystallizes in the pentahemihydrate form (WO2003/059345 and WO2007/056546), that is of therapeutic use.

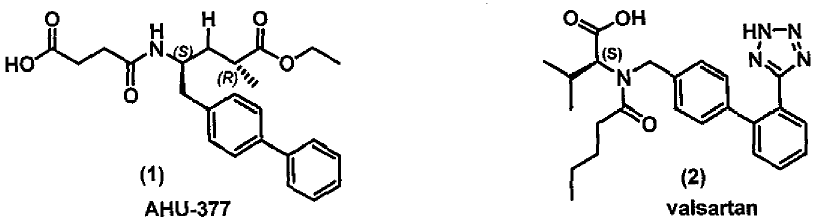

This supramolecular complex has been approved by the Food and Drug Administration (FDA) for the treatment of hypertension and heart failure.

The invention relates to new crystalline salts of AHU-377 with certain amines as defined by the claims, a new method of isolation and purification of these salts incl. a method of obtaining solid forms of AHU-377, applicable for the production of drugs for treatment of hypertension and heart failure.

### Background Art

The first solution of chemical synthesis of AHU-377 (**1**) was described in the patent application EP555175 and subsequently also in the specialized literature (J. Med. Chem. 1995, 38, 1689-1700). Other possibilities of chemical synthesis of AHU-377 (**1**), especially of its advanced intermediates are described in the following patent applications: WO2008/0311567, WO2008/083967, WO2009/090251, WO2011/088797, WO2012/025501, WO2012/025502 and WO2014/032627.

The final step of chemical synthesis leading to AHU-377 is represented by the reaction running in accordance with Scheme 1, wherein the amine (**3**) reacts with succinic acid anhydride (EP555175, J. Med. Chem. 1995, 38, 1689-1700). The reaction results in crude AHU-377 in the free carboxylic acid form. A disadvantage of this procedure is the fact that AHU-377 in the free carboxylic acid form does not crystallize and therefore this form is not suitable for isolation or for chemical purification before the preparation of the sodium salt of AHU-377, which is used as a pharmaceutically acceptable form in practice (WO2007/056546).

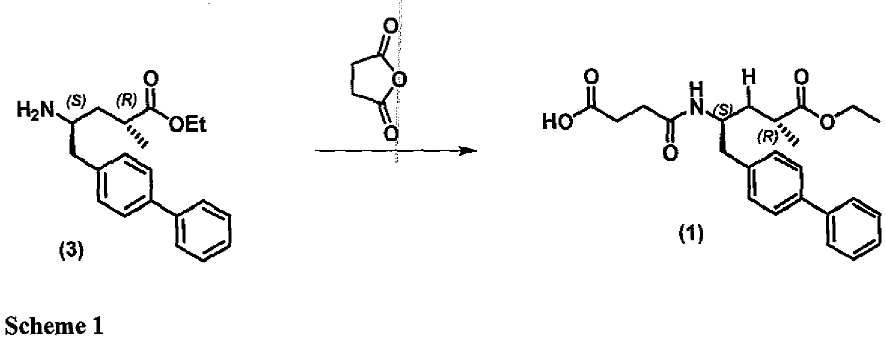

Due to unfavorable isolation characteristics of the free carboxylic acid of AHU-377, complex isolating and purification operations must be used to obtain the sodium salt of AHU-377 in an acceptable quality. The procedure published in the literature may serve as an example of this (J. Med. Chem. 1995, 38, 1689-1700). This procedure is described by Scheme 2. According to this procedure the crude AHU-377 acid is first converted to the respective *tert*-butylester (**4**), then the substance is purified with the use of chromatography, subsequently the pure free acid of AHU-377 is released back, which can be converted to the sodium salt of formula (**5**) by the action of a suitable base, e.g. with the use of sodium hydroxide. Due to the characteristics of the free acid the direct path from the crude acid to the sodium salt could not be used.

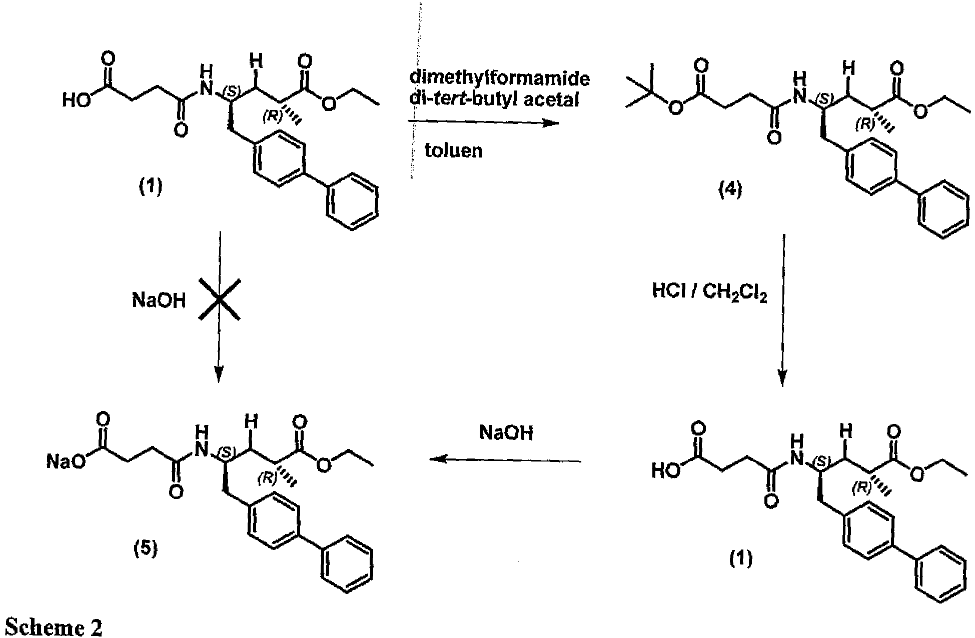

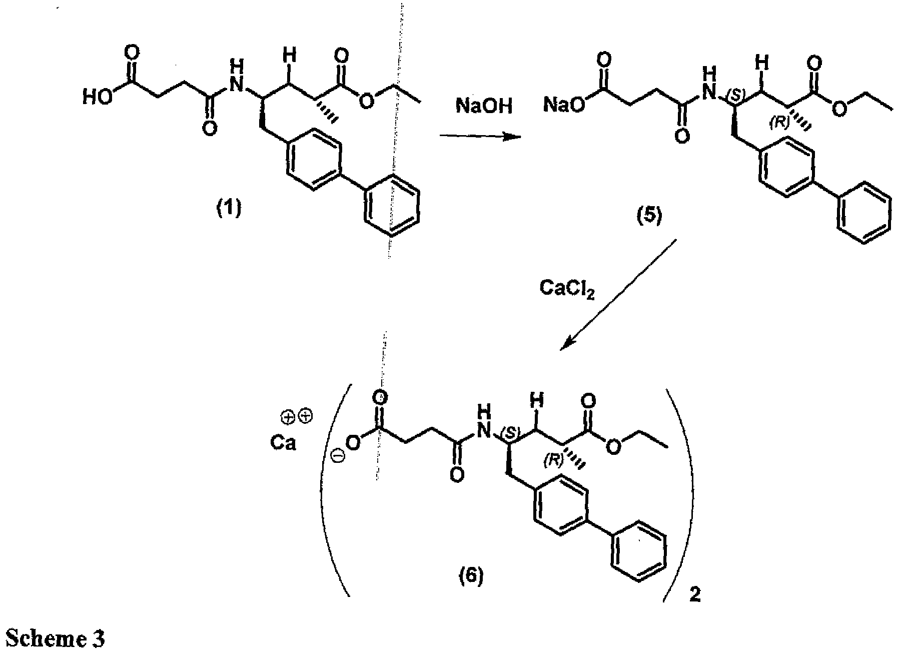

Besides the pharmaceutically acceptable sodium salt of AHU-377 three more pharmaceutically acceptable salts (WO2008/031567, WO20081083967 and WO2003/059345) have been described, namely the calcium salt (**6**), see Scheme 3, the salts with triethanolamine (7) and with tris(hydoxymethyl)aminomethane (**8**), see Scheme 4. Data concerning the chemical purity of these salts, their crystalline structure and the yields of their preparation have not been published yet.

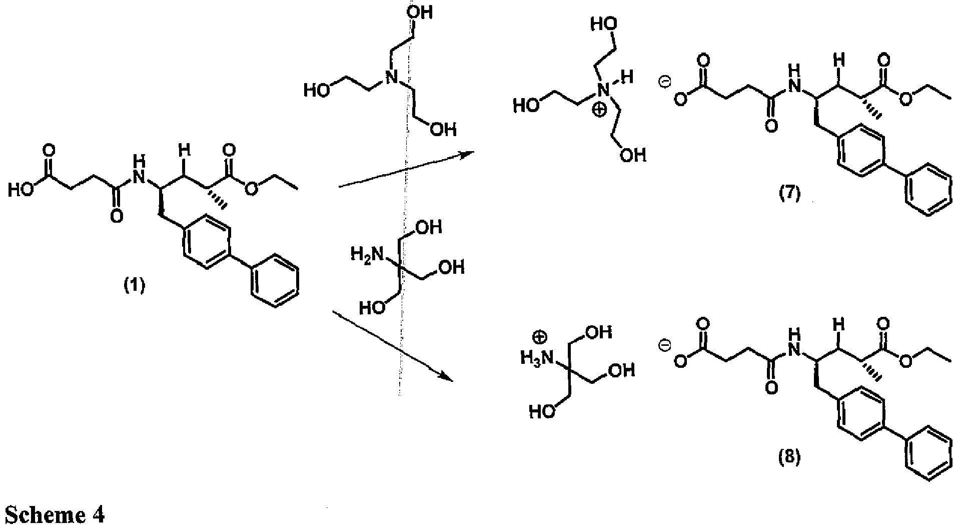

The present invention represents a new, highly efficient and industrially usable method of isolation and purification of AHU-377 based on crystalline salts of AHU-377 with amines.

### Disclosure of Invention

The invention is based on finding well crystallizing forms of AHU-377 that are easy to isolate and chemically purify, subsequently converting them to pharmaceutically acceptable solid forms, especially the sodium and calcium salts of AHU-377. Preparation, as used herein, refers to chemical transformation, purification as well as isolation of the desired substance. Solid forms of AHU-377, especially the crystalline ones, can be of great technological and economical significance as they make it possible to obtain a substance applicable for pharmaceutical purposes.

Chemical synthesis of AHU-377 leads to the free crude carboxylic acid of an oily character (see Scheme 1), which is difficult to isolate in the pure form, it being the consequence of its inability to crystallize. The invention relates to a new and efficient method of isolation and purification of AHU-377, wherein a solution of the AHU-377 carboxylic acid is treated with a suitable amine to provide a solution of salts of AHU-377 with amines (**9**). It has been found out that only some suitable salts of AHU-377 crystallize surprisingly easily, especially with the amines specified below, which can thus be conveniently isolated from the solution in a solid and chemically pure form.

The invention further relates to a convenient method of removal of chemical impurities by repeated crystallizations of salts of AHU-377 with certain amines and to a new preparation method of the amorphous form of the sodium salt, crystalline or semicrystalline calcium salt of AHU-377 using a direct transformation of the salts of AHU-377 with amines by the procedure in accordance with Scheme 5. It has been experimentally found out that a good crystallization capability of amine salts of AHU-377 is not a general characteristics of these salts. The salts of AHU-377 with the following amines have proved their ability to crystallize well and to improve the quality of the isolated substances: *tert*-butylamine, *iso*-propylamine, cyclohexylamine, *n*-propylamine and dicyclohexylamine. The formerly described salts with triethanolamine (**7**) and tris(hydroxmethyl)aminomethane (**8**) can also be obtained in a crystalline form, but their purification ability is worse, as shown in Table 1. In the case of many other amines no crystalline product could be obtained, which namely holds good for di-*iso*-propylamine, di-n-propylamine, di-iso-propylethylamine, triethylamine, nicotinamide, L-arginine, diethylamine, *N*,*N*'-dibenzylethylenediamine and diethanolamine. The best results (yield, chemical purity) were achieved by the new isolation process in the case of AHU-377 salts with primary amines that were characterized by a branched alkyl, e.g. cyclohexylamine, *iso*-propylamine, or *tert*-butylamine.

The process described by Scheme 5 leads to salts of AHU-377 with alkali metals (**10**), or alkaline earth metals (**11**) in four steps. Alternatively, salts can be obtained from the re-released acid (**1**), which is converted to a salt with alkali metals (**10**), or alkaline earth metals (**11**) with the use of alkali metal or alkaline earth metal alcoholates, or salts of organic acids derived from the above mentioned metals (Scheme 6). The procedure of individual steps is described in a more detailed way below.

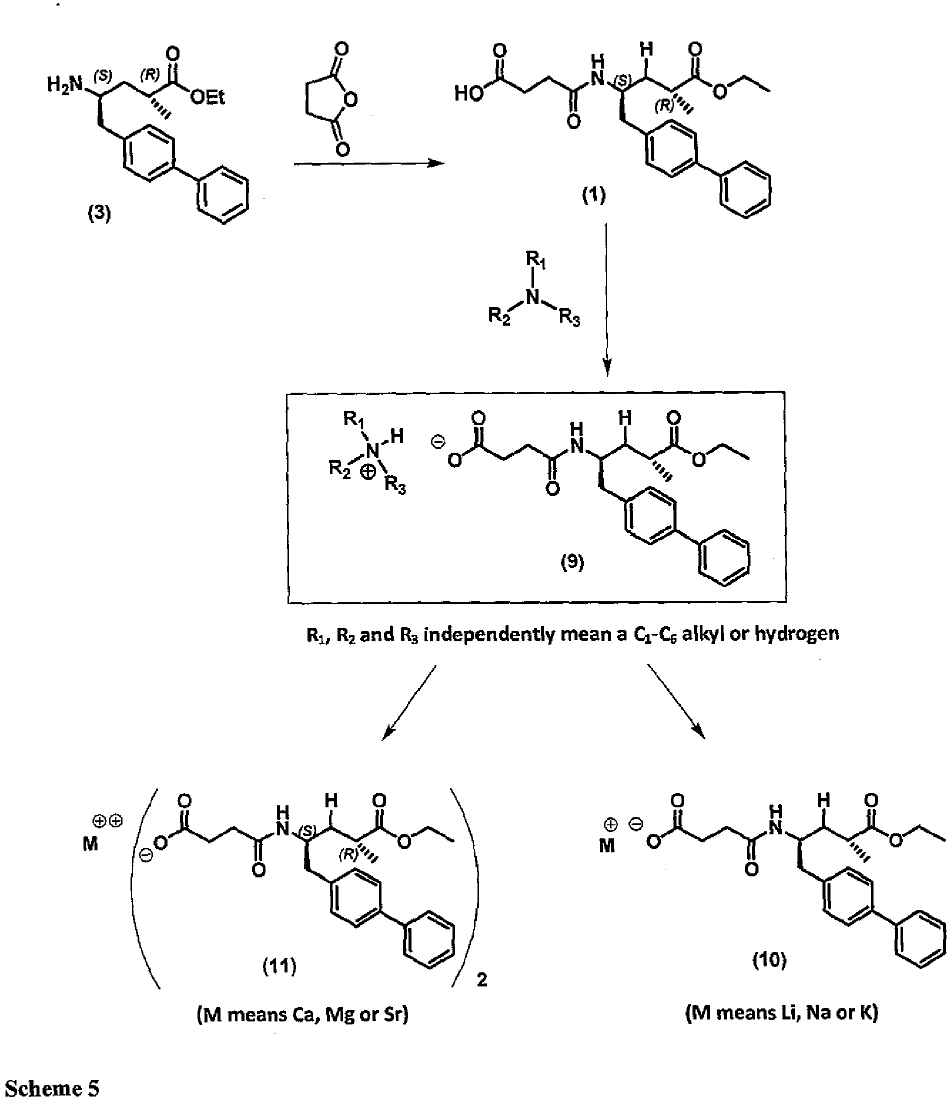

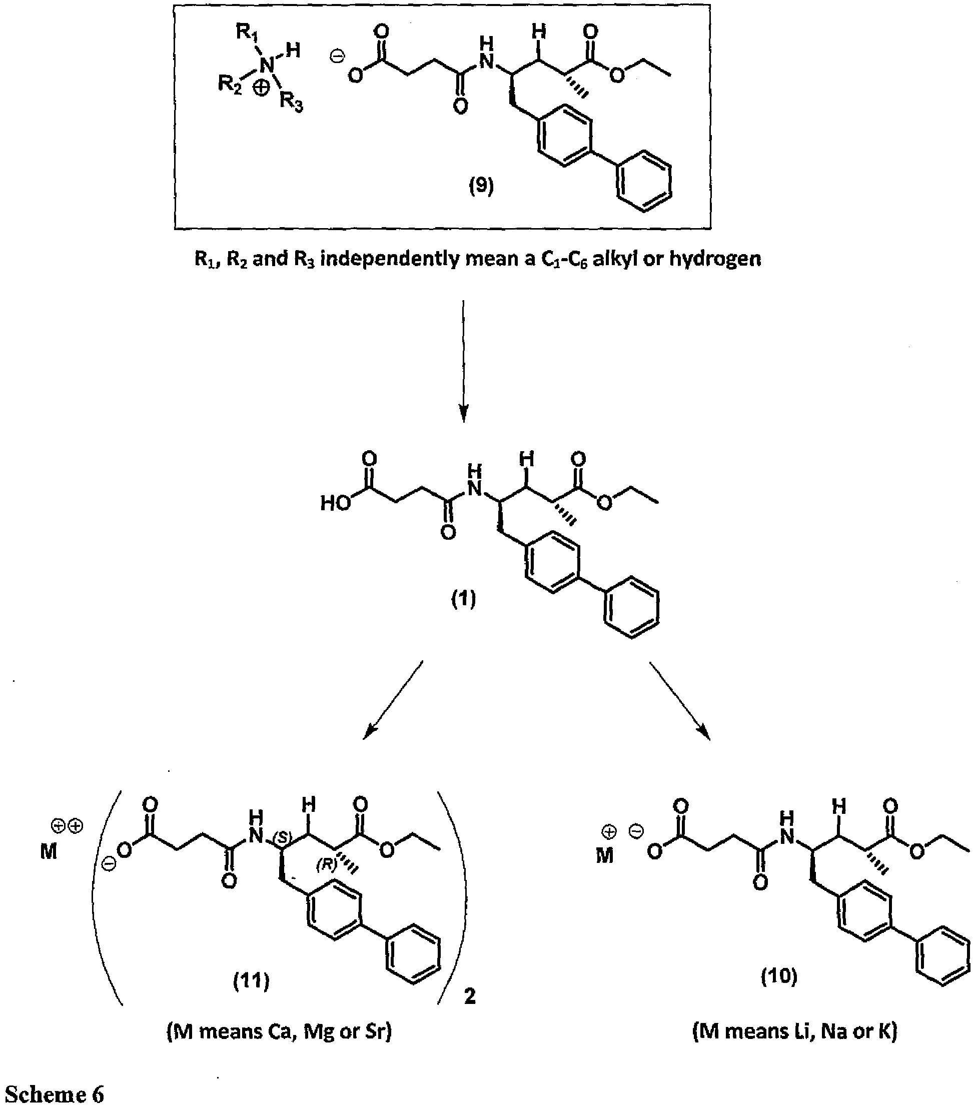

### ► Step 1. - Chemical synthesis of AHU-377 acid

The first step represents chemical synthesis of AHU-377, especially the final reaction of the amine (**3**) with succinic acid anhydride in accordance with Scheme 5. The output of this step is crude AHU-377 acid, which also contains undesired constituents such as residual solvents and residues of reactants, mainly derivatives of succinic acid. The product of the first step, which failed to be obtained in a solid form, usually contained 97.5 to 98.5 % of the target compound according to HPLC analyses.

### ► Step 2. - Preparation and isolation of crystalline salts of AHU-377 with amines

The second step represents isolation of AHU-377 in the form of solid, well crystallizing salts of AHU-377 with amines (**9**). Preparation of these salts consists in dissolution of AHU-377 acid in at least one organic solvent, adding of a suitable amine to the solution, agitation of the mixture, filtration of the separated solid form and drying of the isolated substance. The output of this step is a crystalline form of a salt of AHU-377 with an amine that is characterized by a higher chemical purity as compared to the input AHU-377 acid.

Crystalline salts of AHU-377 with primary amines selected from the group of *tert*-butylamine, *iso*-propylamine and cyclohexylamine especially offer convenient crystallizing characteristics, which are associated with the crystal structure of these salts. These salts have been found out to provide stable crystal forms that can be unambiguously characterized by means of X-ray powder diffraction (XRPD, see analytic methods and annexes). The contribution of the discovered process of purification of AHU-377 in the form of its salts with amines is an exceptionally good ability of these salts to crystallize from solutions of organic solvents, which leads to increased chemical purity of the isolated substance.

Organic solvents usable for isolation of salts of AHU-377 with amines can be selected from the group: esters of C1-C5 organic acids with C1-C6 alcohols, C1-C6 alcohols, C3-C6 ketones, C4-C6 ethers, C1-C6 chlorinated hydrocarbons, liquid aromatic hydrocarbons, C1-C7 alkanes, C1-C7 cycloalkanes. The following solvents or their mixtures are especially suitable: ethyl acetate, *iso*-propyl acetate, methanol, ethanol, *iso*-propyl alcohol, acetone, methyl ethyl ketone, diethyl ether, tetrahydrofuran, 2-methyltetrahydrofuran, *tert*-butyl methyl ether, dichloromethane, toluene, xylenes, hexane, heptane, cyclohexane. The best results were achieved with ethyl acetate or *iso*-propyl acetate.

An especially convenient method of isolation of a salt of an amine with AHU-377 is one where steps 1 and 2 are joined together, i.e. a method that does not require isolation of AHU-377 acid. This modification is characterized by isolation of the salt of AHU-377 with a primary amine directly from the reaction environment used for performing step 1. In this case, the mixture does not have to be concentrated after the initial reaction and then the crude AHU-377 acid does not need to be dissolved in another solvent, see Example 4.

### ► Step 3. - Recrystallization of salts of AHU-377 with amines

The third step is crystallization of the salt of AHU-377 with an amine (**9**) from at least one organic solvent to further increase the chemical purity, wherein the obtained substance exhibits a higher HPLC purity than 99.5%, the contents of individual impurities being less than 0.1%. This step represents the final removal of chemical impurities. The output of this step is a chemically pure crystalline salt of AHU-377 with an amine in a pharmaceutically acceptable quality.

Organic solvents usable for crystallization of salts of AHU-377 with amines can be selected from the group: esters of C1-C5 organic acids with C1-C6 alcohols, C1-C6 alcohols, C3-C6 ketones, C4-C6 ethers, C1-C6 chlorinated hydrocarbons, liquid aromatic hydrocarbons, C1-C7 alkanes, C1-C7 cycloalkanes. The following solvents or their mixtures are especially suitable: ethyl acetate, *iso*-propyl acetate, methanol, ethanol, *iso*-propyl alcohol, acetone, methyl ethyl ketone, diethyl ether, tetrahydrofuran, 2-methyltetrahydrofuran, *tert*-butyl methyl ether, dichloromethane, toluene, xylenes, hexane, heptane, cyclohexane. The best results were achieved with ethyl acetate or *iso*-propyl acetate.

### ► Step 4. - Conversion of salts of AHU-377 with amines to salts of AHU-377 with metals

The last step relates to the process of conversion of crystalline salts of AHU-377 with amines (**9**) to a pharmaceutically acceptable salt with a metal. Depending on the conditions these may be salts of AHU-377 with alkali metals from the group of lithium, sodium or potassium (**10**, M = Li, Na, K), or salts of AHU-377 with alkaline earth metals from the group of calcium, magnesium or strontium (**11**, M = Ca, Mg, Sr). The source of the metal can be the respective inorganic and organic salts, alcoholates or hydroxides of these metals. A pharmaceutically acceptable form of AHU-377 is the sodium or calcium salt. The output of this step is an active pharmaceutical substance (API) usable for the preparation of a drug for the treatment of hypertension and heart failure.

A convenient aspect of preparation of the amorphous form of the sodium salt of AHU-377 is a direct conversion of salts of AHU-377 with amines to this form, which has not been described yet. The discovered method of preparation of the amorphous form of the sodium salt of AHU-377 is especially convenient through the use of crystalline and chemically pure salts of AHU-377 with primary amines, which, compared to secondary amines, are characterized by lower basicity and sufficient volatility, which enables efficient removal of the primary amine without undesired contamination of the product during the preparation of the sodium salt of AHU-377. Analogously to the preparation of the sodium salt of AHU-377, crystalline salts of AHU-377 with primary amines can be conveniently used for the preparation of a crystalline or semicrystalline form of the calcium salt of AHU-377.

This invention provides especially processes concerning the performance of isolation and chemical purification of AHU-377, i.e. steps 2 and 3. It further provides processes of preparation of the amorphous form of the sodium salt, crystalline and semicrystalline form of the calcium salt of AHU-377, which are based on using the salts of AHU-377 with amines, i.e. step 4. The newly discovered crystalline forms of AHU-377 with amines and the method of conversion of these forms to salts of AHU-377 with metals can be conveniently applied in the industrial scale and a pharmaceutical substance complying with stringent quality criteria can be effectively obtained.

According to Scheme 6, salts of AHU-377 with alkali metals (**10**), or alkaline earth metals (**11**) can be alternatively obtained from the re-released free acid of AHU-377 (**1**), which is converted to the salts of (**10**) or (**11**) in organic solvents by the action of suitable agents. A suitable source of the metal can be the respective alcoholates, preferably sodium methoxide, potassium methoxide, sodium ethoxide, potassium ethoxide, sodium *iso*-propoxide, potassium iso-propoxide, soidum *tert*-butoxide, or potassium *tert*-butoxide. Salts of organic acids, preferably salts of organic acids soluble in organic solvents, can also be used instead of the alcoholates. For example, the sodium, potassium or calcium salts of 2-ethylhexanoic acid can be used as salts of organic acids.

Herein described are pharmaceutically acceptable crystalline and semicrystalline forms of the calcium salt of sacubitril and methods for the preparation thereof. These solid forms of the calcium salt of sacubitril are prepared by crystallization, precipitation or evaporation from a solution of the calcium salt of sacubitril in a suitable solvent or mixtures of solvents.

Herein described is a crystalline form I of the calcium salt of sacubitril, exhibiting the following characteristic reflections in the X-ray powder pattern with the use of CuKα radiation: 3.6; 6.4; 14.5 and 19.6 ± 0.2° 2-theta. In some disclosures crystalline form I of the calcium salt of sacubitril is characterized by the following other reflections in the X-ray powder pattern: 8.4; 10.8; 17.7; 20.6 and 22.5 ° ± 0.2° 2-theta. In some embodiments crystalline form I of the calcium salt of sacubitril is further characterized by the differential scanning calorimetry curve with the melting point at 86°C.

Herein described is a method for the preparation of crystalline form I of the calcium salt of sacubitril, comprising:
a/ dissolution of the calcium salt of sacubitril in one of the solvents selected from the group of: ethanol, methanol, dimethyl sulfoxide, butyl acetate, acetonitrile, isopropyl alcohol, butanol acetone, or a mixture of these solvents at a temperature in the range from 25°C to the boiling point of the solvent;
b/ separation of the solid fraction by crystallization or precipitation at temperatures in the range of -30°C to the boiling point of the solvent or by evaporation from the solution;
c/ isolation of the separated solid fraction.

Herein described is a crystalline form II of the calcium salt of sacubitril, exhibiting the following characteristic reflections in the X-ray powder pattern with the use of CuKα radiation: 3.2; 7.4; 12.9; 18.7 and 22.5; ± 0.2° 2-theta. In some disclosures crystalline form II of the calcium salt of sacubitril is characterized by the following other reflections in the X-ray powder pattern: 11.4; 16.2; 23.7 and 27.1 ± 0.2° 2-theta. In some disclosures crystalline form II of the calcium salt of sacubitril is further characterized by the differential scanning calorimetry curve with the melting point at 68°C.

Further described herein is a method for the preparation of crystalline form II of the calcium salt of sacubitril, comprising:
a/ dissolution of the calcium salt of sacubitril in a dioxane:water (1:1) mixture at a temperature in the range from 25°C to the boiling point of the solvent;
b/ separation of the solid fraction by crystallization or precipitation at temperatures in the range of -30°C to the boiling point of the solvent or by evaporation from the solution;
c/ isolation of the separated solid fraction.

Further described herein is a semicrystalline form III of the calcium salt of sacubitril, exhibiting the following reflections in the X-ray powder pattern with the use of CuKα radiation: 4.0 and 4.7 ± 0.2° 2-theta. In some disclosures semicrystalline form III of the calcium salt of sacubitril is further characterized by the differential scanning calorimetry curve with the melting point at 124°C.

Further described herein is a method for the preparation of semicrystalline form III of the calcium salt of sacubitril, comprising:
a/ dissolution of the calcium salt of sacubitril in one of the solvents selected from the group of: tetrahydrofuran, toluene, xylene, dichloromethane, or a mixture of these solvents at a temperature in the range from 25°C to the boiling point of the solvent;
b/ separation of the solid fraction by crystallization or precipitation at temperatures in the range of -30°C to the boiling point of the solvent or by evaporation from the solution;
c/ isolation of the separated solid fraction.

Further described herein is a semicrystalline solid form IV of the calcium salt of sacubitril, exhibiting the following characteristic reflections in the X-ray powder pattern with the use of CuKα radiation: 4.6; 6.4 and 14.3 ± 0.2° 2-theta. In some disclosures semicrystalline solid form IV of the calcium salt of sacubitril is further characterized by the differential scanning calorimetry curve with the melting point at 173°C.

Further described herein is a method for the preparation of semicrystalline form IV of the calcium salt of sacubitril, comprising:
a/ dissolution of the calcium salt of sacubitril in chloroform at a temperature in the range from 25°C to the boiling point of the solvent;
b/ separation of the solid fraction by crystallization or precipitation at temperatures in the range of -30°C to the boiling point of the solvent;
c/ isolation of the separated solid fraction.

Salts of pharmaceutically active substances generally have higher solubility and biological availability values than their corresponding forms of free acids or bases.

Although preparation of a salt by a reaction of an acid and base is a well-known method, it is always a problem to obtain the required salts in the solid phase and purity corresponding to the demands for their pharmaceutical use. Biological availability greatly depends on whether a crystalline or amorphous product is obtained. An amorphous product is usually more readily soluble, it cannot often be obtained in the required quality and it is also often unstable. Conversely, compared to the amorphous form, a crystalline product is often stable, its required purity is easier to achieve and it dissolves more slowly. Mixtures of the amorphous and crystalline solid phase (semicrystalline forms) may represent a solution to the problem.

Described herein are several solid forms of the calcium salt of sacubitril in a crystalline form or in a mixture of a crystalline and amorphous form.

Described herein are solid forms of the calcium salt of sacubitril in variable molar ratios. In the invention, 2:1 molar ratios are preferred.

Solid forms of the calcium salt of sacubitril can be prepared in adequate ratios and yields with high chemical purity in a crystalline or amorphous form, or in a mixture of an amorphous and crystalline solid phase.

These solid forms can be both anhydrous and/or non-solvated, and they can have the form of hydrates/solvates of the respective solvents.

These solid forms are suitable for preparation of sacubitril with a high chemical and optical purity. The preparation of the novel solid form of sacubitril consists in a reaction of the cyclohexylamine salt of sacubitril with calcium chloride in water, or in a mixture of water with a suitable organic solvent. The resulting product of this reaction is recrystallized in a suitable solvent, which can be ketones, esters, ethers, amides, nitriles or organic acids, alcohols, aliphatic and aromatic hydrocarbons, chlorinated hydrocarbons, water or mixtures thereof. Aliphatic C1-C4 alcohols, ethers, chlorinated solvents, water, or mixtures thereof are preferred. The most frequently used solvents are ethanol, tetrahydrofuran, dioxane, chloroform, water or mixtures thereof.

The final product is typically precipitated or crystallized at temperatures in the range of -30°C to the boiling point of the solvent.

Described herein are crystalline forms of the calcium salt of sacubitril (form I and form II), which, due to their crystalline nature, have excellent physical and chemical stability. The prepared solid forms of the calcium salts of sacubitril exhibit a sufficiently high melting point, physical and chemical stability for use in a dosage form. The main advantage of the calcium salts of sacubitril over the sodium salt of sacubitril is their higher physical and chemical stability during storage.

A sample of the calcium salt of sacubitril (form I) was exposed to the temperature of 80°C at 0% relative humidity (RH) and 75% RH for 3 days. The crystalline state of the calcium salt of sacubitril did not change during this time period. The same experiment was also conducted with the sodium salt of sacubitril, which exhibited a high rate of polyform instability and at 75% RH the sample became completely amorphous (see Table 1).

**Table 1: Stability of solid forms of sacubitril salts**

| **sample** | **X-ray powder analysis** | **Result after loading of the sample at 80°C/0% RH** | **Result after loading of the sample at 80°C/75% RH** |
|---|---|---|---|
| calcium salt of sacubitril | Crystalline sample (form I) | Crystalline sample (form I)I | Crystalline sample (form I)I |
| sodium salt of sacubitril | Semicrystalline phase | Semicrystalline phase | Amorphous sample |

The calcium salt of sacubitril has been found not to change its chemical purity under the test conditions.

Out of the described preparation processes of solid forms of the calcium salt of sacubitril the process of preparation from ethanol is preferred as the prepared crystalline product exhibits suitable stability for use in the dosage form. Crystalline forms of the calcium salt of sacubitril can be prepared and isolated with a high yield and high chemical purity.

The cyclohexyl ammonium salt of sacubitril was prepared according to the procedure presented in Example 1.

The calcium salt of sacubitril was prepared according to the procedure presented in Example 2.

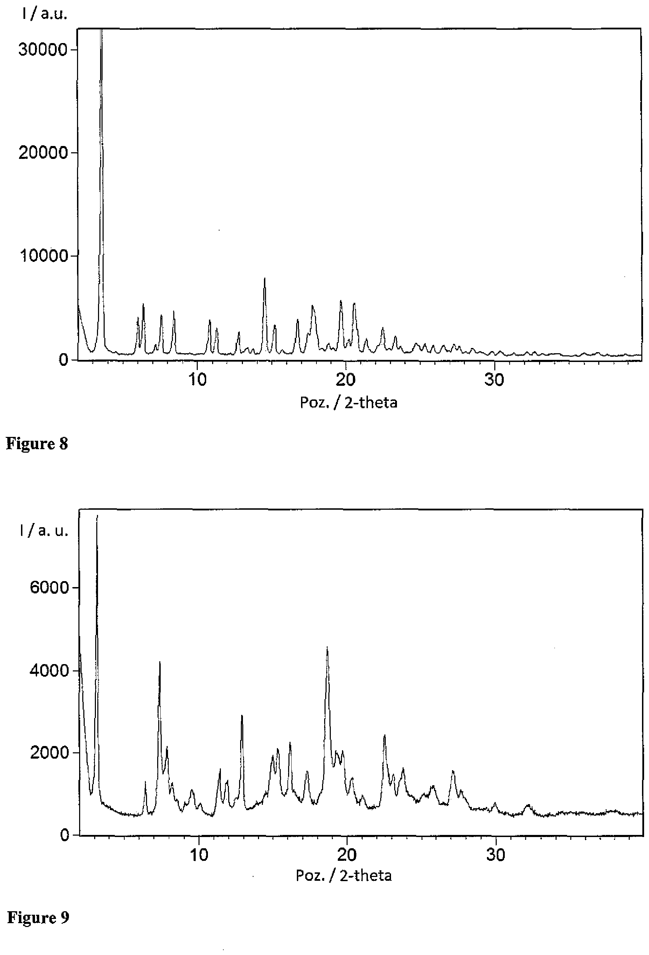

Crystalline form I of the calcium salt of sacubitril is characterized by the reflections presented in **Table 2**. Table 2 includes reflections whose relative intensity value is higher than 1 %. Characteristic diffraction peaks of crystalline form I of the calcium salt of sacubitril according to the present invention with the use of CuKα radiation are: 3.6; 6.4; 14.5 and 19.6 ± 0.2° 2-theta. The X-ray powder pattern is shown in **Fig. 8**.

**Table 2**

| Position [°2Th.] | Interplanar spacing [Å] [Å]=0.1nm | Rel. intensity (%) |
|---|---|---|
| 3.58 | 24.640 | 100.0 |
| 5.99 | 14.734 | 9.3 |
| 6.35 | 13.899 | 12.4 |
| 7.19 | 12.279 | 2.3 |
| 7.56 | 11.680 | 9.8 |
| 8.40 | 10.514 | 10.6 |
| 10.78 | 8.202 | 8.8 |
| 11.27 | 7.848 | 6.7 |
| 12.75 | 6.940 | 5.6 |
| 14.51 | 6.101 | 19.7 |
| 15.16 | 5.842 | 7.7 |
| 16.70 | 5.305 | 8.3 |
| 17.73 | 4.999 | 11.1 |
| 18.82 | 4.711 | 2.5 |
| 19.66 | 4.513 | 13.1 |
| 20.17 | 4.399 | 3.0 |
| 20.55 | 4.318 | 12.4 |
| 21.36 | 4.157 | 3.4 |
| 22.45 | 3.957 | 6.3 |
| 23.31 | 3.813 | 4.4 |
| 24.71 | 3.601 | 2.3 |
| 25.30 | 3.517 | 2.4 |
| 25.87 | 3.441 | 2.1 |
| 26.57 | 3.352 | 2.0 |
| 27.27 | 3.268 | 2.4 |
| 27.63 | 3.226 | 1.8 |
| 28.52 | 3.127 | 1.4 |
| 29.80 | 2.996 | 0.9 |
| 30.35 | 2.942 | 1.1 |

The melting point of crystalline form I of the calcium salt of sacubitril as measured by differential scanning calorimetry (DSC) was 86°C.

Crystalline form II of the calcium salt of sacubitril is characterized by the reflections presented in **Table 3**. Table 3 includes reflections whose relative intensity value is higher than 1 %. Characteristic diffraction peaks of crystalline form II of the calcium salt of sacubitril according to the present invention with the use of CuKα radiation are: 3.2; 7.4; 12.9; 18.7 and 22.5; ± 0.2° 2-theta. The X-ray powder pattern is shown in **Fig. 9**.

**Table 3**

| Position [°2Th.] | Interplanar spacing [Å] [Å]=0.1nm | Rel. intensity (%) |
|---|---|---|
| 3.19 | 27.667 | 100.0 |
| 6.43 | 13.737 | 12.6 |
| 7.40 | 11.943 | 57.1 |
| 7.85 | 11.255 | 22.7 |
| 9.52 | 9.279 | 9.8 |
| 10.09 | 8.758 | 4.5 |
| 11.38 | 7.771 | 15.0 |
| 11.89 | 7.439 | 12.1 |
| 12.90 | 6.857 | 34.4 |
| 14.95 | 5.923 | 16.6 |
| 15.34 | 5.770 | 17.9 |
| 16.16 | 5.481 | 22.7 |
| 17.27 | 5.130 | 11,9 |
| 18.67 | 4.748 | 57.8 |
| 19.31 | 4.592 | 17.4 |
| 19.73 | 4.495 | 13.9 |
| 20.33 | 4.364 | 9.7 |
| 22.55 | 3.940 | 25.8 |
| 23.04 | 3.858 | 10.3 |
| 23.75 | 3.744 | 14.1 |
| 25.19 | 3.504 | 6.1 |
| 25.81 | 3.449 | 5.0 |
| 27.08 | 3.290 | 12.5 |
| 27.70 | 3.218 | 6.6 |
| 29.92 | 2.984 | 4.2 |
| 32.13 | 2.783 | 4.3 |

The melting point of crystalline form II of the calcium salt of sacubitril as measured by differential scanning calorimetry (DSC) was 68°C.

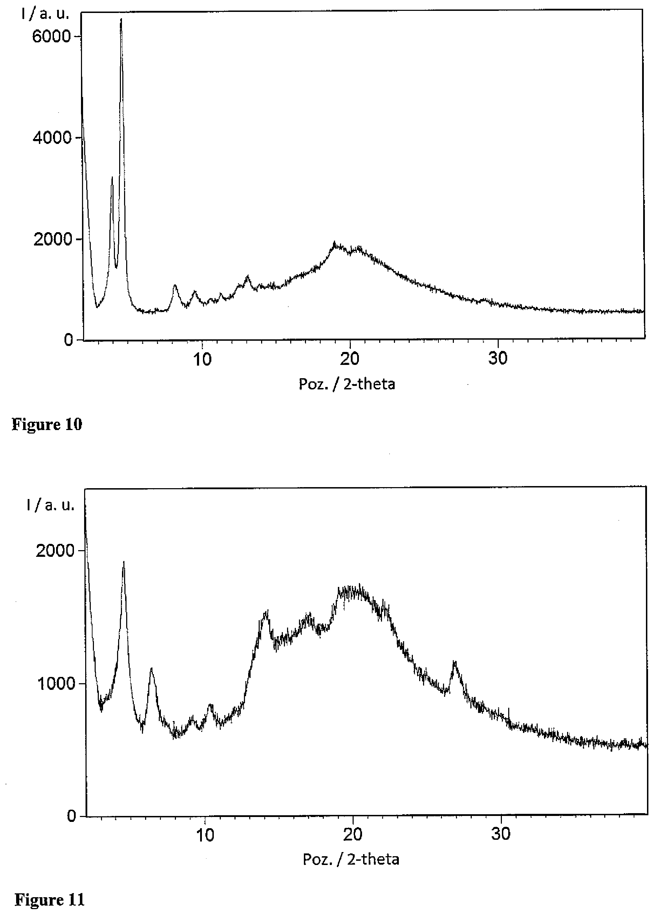

Semicrystalline form III of the calcium salt of sacubitril is characterized by the reflections presented in **Table 4**. Table 4 includes reflections whose relative intensity value is higher than 1 %. Characteristic diffraction peaks of semicrystalline form III of the calcium salt of sacubitril according to the present invention with the use of CuKα radiation are: 4.0 and 4.7 ± 0.2° 2-theta. The X-ray powder pattern is shown in **Fig. 10**.

**Table 4**

| Position [°2Th.] | Interplanar spacing [Å] [Å]=0.1nm | Rel. (%) |
|---|---|---|
| 3.19 | 27.667 | 100.0 |
| 6.43 | 13.737 | 12.6 |
| 7.40 | 11.943 | 57.1 |
| 7.85 | 11.255 | 22.7 |
| 9.52 | 9.279 | 9.8 |
| 10.09 | 8.758 | 4.5 |
| 11.38 | 7.771 | 15.0 |
| 11.89 | 7.439 | 12.1 |
| 12.90 | 6.857 | 34.4 |
| 14.95 | 5.923 | 16.6 |
| 15.34 | 5.770 | 17.9 |
| 16.16 | 5.481 | 22.7 |
| 17.27 | 5.130 | 11,9 |
| 18.67 | 4.748 | 57.8 |
| 19.31 | 4.592 | 17.4 |
| 19.73 | 4.495 | 13.9 |
| 20.33 | 4.364 | 9.7 |
| 22.55 | 3.940 | 25.8 |
| 23.04 | 3.858 | 10.3 |
| 23.75 | 3.744 | 14.1 |
| 25.19 | 3.504 | 6.1 |
| 25.81 | 3.449 | 5.0 |
| 27.08 | 3.290 | 12.5 |
| 27.70 | 3.218 | 6.6 |
| 29.92 | 2.984 | 4.2 |
| 32.13 | 2.783 | 4.3 |

The melting point of semicrystalline form III of the calcium salt of sacubitril as measured by differential scanning calorimetry (DSC) was 124°C.

Semicrystalline form IV of the calcium salt of sacubitril is characterized by the reflections presented in **Table 5**. Table 5 includes reflections whose relative intensity value is higher than 1 %. Characteristic diffraction peaks of semicrystalline form IV of the calcium salt of sacubitril according to the present invention with the use of CuKα radiation are: 4.6, 6.4 and 14.3 ± 0.2° 2-theta. The X-ray powder pattern is shown in **Fig. 11**.

**Table 5**

| Position [°2Th.] | Interplanar spacing [Å] [Å]=0.1nm | Rel. intensity (%) |
|---|---|---|
| 4.58 | 19.270 | 100.0 |
| 6.42 | 13.762 | 39.9 |
| 9.26 | 9.542 | 6.3 |
| 10.33 | 8.553 | 13.4 |
| 14.28 | 6.199 | 35.4 |
| 17.02 | 5.205 | 12.2 |
| 19.10 | 4.644 | 23.9 |
| 22.28 | 3.988 | 22.9 |
| 26.92 | 3.310 | 23.6 |

The melting point of semicrystalline form IV of the calcium salt of sacubitril as measured by differential scanning calorimetry (DSC) was 173°C.

The solid forms of calcium salts of sacubitril prepared according to this invention can be used for the preparation of pharmaceutical compositions, especially solid dosage forms, e.g. tablets or capsules. Such pharmaceutical compositions can only contain at least one excipient from the group of fillers (e.g. lactose), binders (e.g. microcrystalline cellulose), disintegrants (e.g. sodium salt of croscarmellose), lubricants (e.g. magnesium stearate), surfactants etc. The calcium salt of sacubitril can be mixed with the above mentioned excipients, screened through a sieve and the resulting mixture can be tabletted or filled into capsules. The tablets can be further coated with common coating compounds, e.g. polyvinyl alcohol or polyethylene glycol.

### Brief Description of Drawings

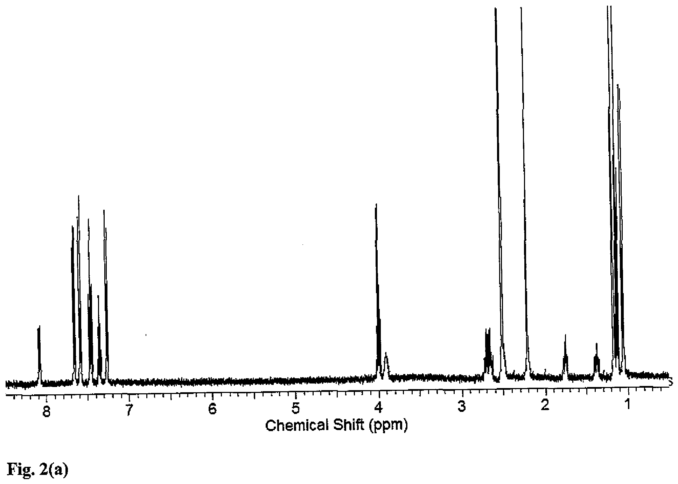

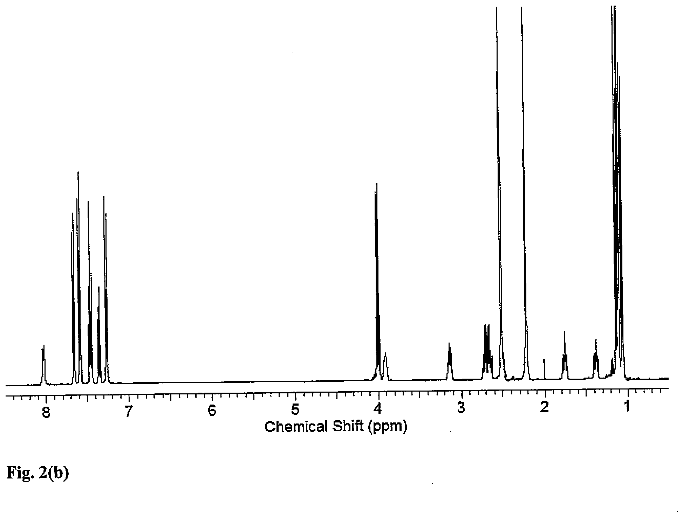

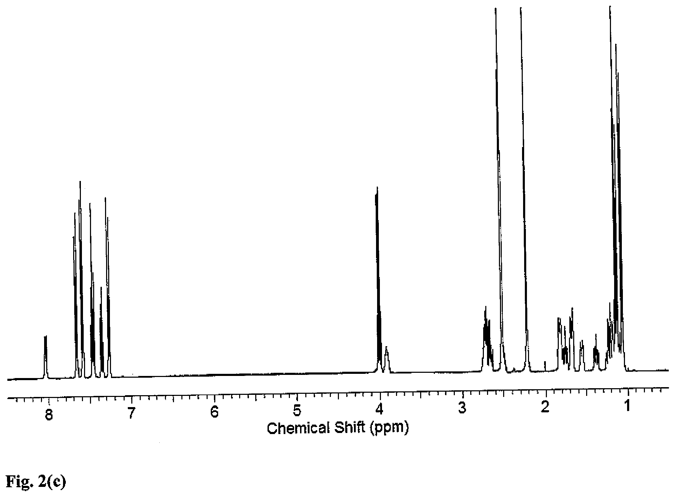

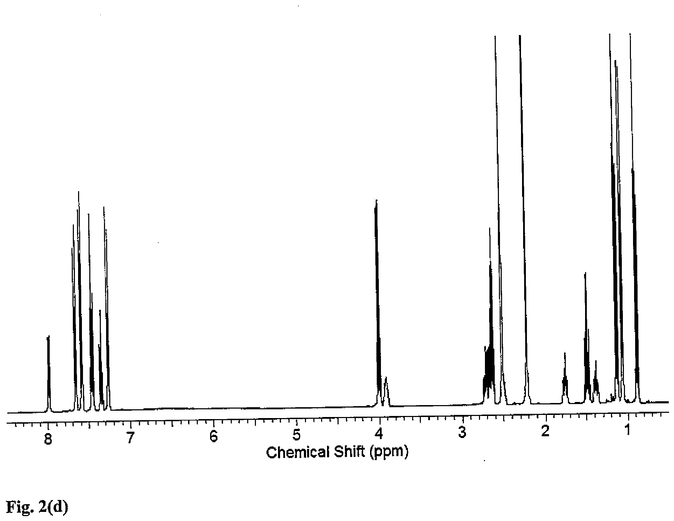

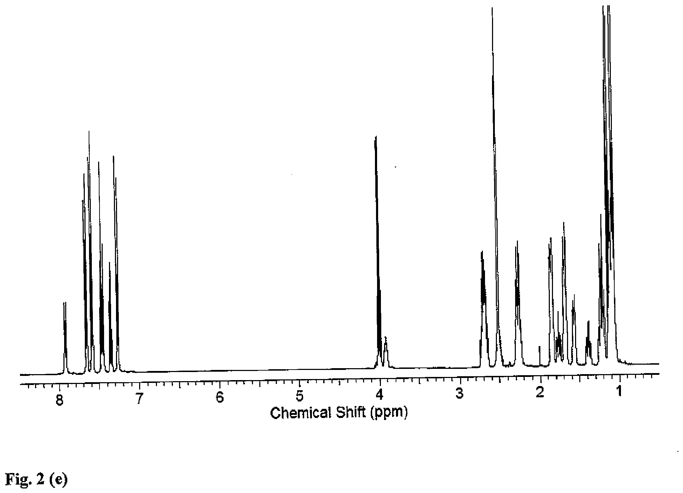

**Fig. 1****:** XRPD pattern of crystalline salts of AHU-377 with amines, i.e. dependence of diffused light intensity on the 2-theta diffraction angle.
   (a) salt with *tert*-butylamine
   (b) salt with *iso*-propylamine
   (c) salt with cyclohexylamine
   (d) salt with n-propylamine
   (e) salt with dicyclohexylamine
**Fig. 2**: ¹H NMR spectra of crystalline salts of AHU-377 with amines.
   (a) salt with *tert*-butylamine
   (b) salt with *iso*-propylamine
   (c) salt with cyclohexylamine
   (d) salt with n-propylamine
   (e) salt with dicyclohexylamine
**Fig. 3****:** XRPD pattern of the crystalline salt of AHU-377 (**11**, M stands for calcium). The pattern illustrates dependence of diffused light intensity on the 2-theta diffraction angle.
**Fig. 4****:** XRPD pattern semicrystalline calcium salt of AHU-377 (**11**, M stands for calcium). The pattern illustrates dependence of diffused light intensity on the 2-theta diffraction angle.
**Fig. 5****:** XRPD pattern of the crystalline tris(hydroxmethyl)aminomethane salt of AHU-377 (**8**). The pattern illustrates dependence of diffused light intensity on the 2-theta diffraction angle.
**Fig. 6****:** XRPD pattern of the amorphous sodium salt of AHU-377 (**10**, M stands for sodium). The pattern illustrates dependence of diffused light intensity on the 2-theta diffraction angle.
**Fig. 7****:** ¹H NMR spectrum of the sodium salt of AHU-377 (**10**, M stands for sodium).
**Fig. 8**: XRPD pattern of sacubitril calcium salt crystalline Form I.
**Fig. 9****:** XRPD pattern of sacubitril calcium salt crystalline Form II.
**Fig. 10****:** XRPD pattern of sacubitril calcium salt semicrystalline Form III.
**Fig. 11****:** XRPD pattern of sacubitril calcium salt semicrystalline Form IV.

### Examples

The object of the invention will be clarified in a more detailed way in the following examples, which, however, do not have any influence on the invention scope defined in the claims.

### EXAMPLE 1 (synthesis of AHU-377 acid) (Reference Example)

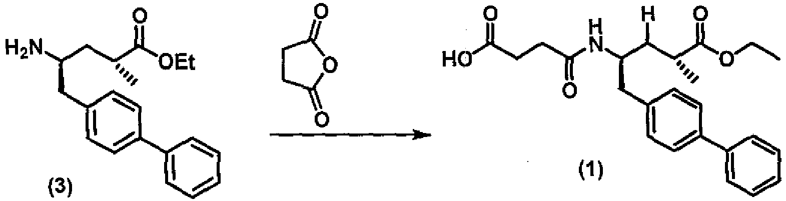

100 ml of dichloromethane and 12 ml of triethylamine was added to 10 g of the hydrochloride of amine (**3**). The mixture was agitated at the room temperature for approx. 10 minutes. This was followed by addition of 4.3 g of succinic anhydride and agitation of the mixture at the room temperature for 4 hours. 100 ml of 1M HCl was added to the mixture, the layers were separated, the organic layer was washed with water and dried over sodium sulphate. After filtration of the desiccant the solvent was evaporated *in vacuo* (75°C, 10 mbar) and the product was obtained in the form of transparent honey. According to HPLC analyses the product usually contained 97.5 to 98.5% of the desired substance.

### EXAMPLE 2 (general method of preparation and isolation of a salt of AHU-377 with amines)

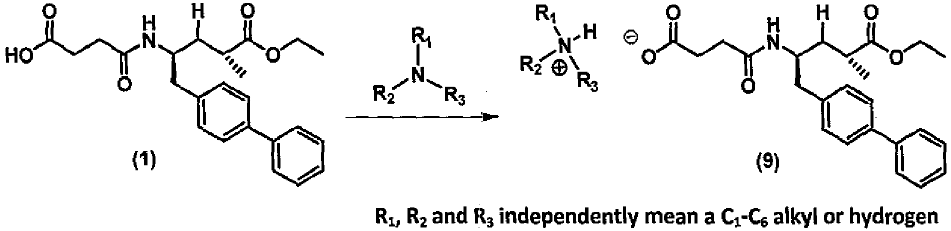

AHU-377 acid (**1**) was dissolved in an organic solvent. An amine was added to the solution in an amount corresponding to 1.1 equivalents of AHU-377. The mixture was agitated. The precipitated crystalline salt of the amine with AHU-377 (**9**) was isolated by filtration, washed with mother liquor and with the solvent used and dried *in vacuo.* The isolated crystalline salts were characterized by means of XRPD, the structure was verified with the use of ¹H NMR and the chemical purity with the use of HPLC. The measured values of chemical purity for the series of amines are summarized in Table 6, the results of other analyses (¹H NMR, XRPD) are included below.

### EXAMPLE 2a (method of preparation and isolation of the crystalline salt of AHU-377 with tert-butylamine)

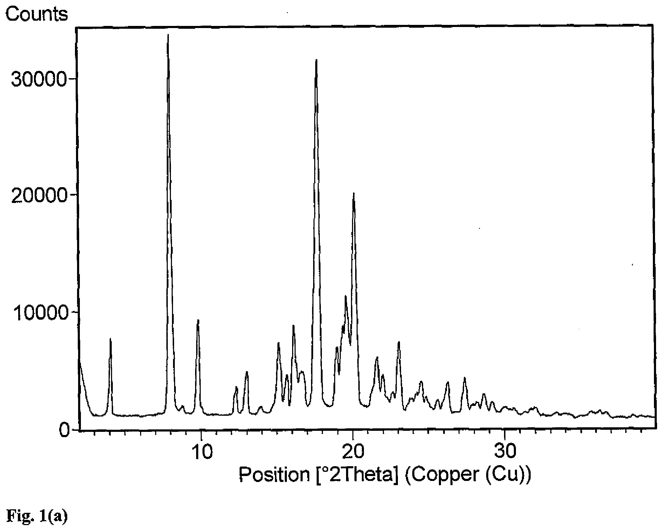

1 g of AHU-377 acid (**1**) was dissolved in 10 ml of ethyl acetate. *Tert*-butylamine was added to the solution in an amount corresponding to 1.1 equivalents of AHU-377. The mixture was agitated. The precipitated crystalline salt of the amine with AHU-377 (**9**) was isolated by filtration, washed with mother liquor and with the solvent used and dried *in vacuo.* The yield of the salt with *tert*.butylamine was 81.5%, the chemical purity according to HPLC was 99.4%. The result of XRPD is presented in Table 7 and in Fig. 1 (a). ¹H NMR (DMSO-D6): 1.06 (d, 3H), 1.13 (t, 3H), 1.17 (s, 9H), 1.38 (m, 1H), 1.76 (m, 1H), 2.22 (s, 3H), 2.24 (m, 1H), 2.68 (m, 2H), 3.91 (m, 1H), 4.00 (q, 2H), 7.27 (d, 2H), 7.35 (t, 1H), 7.46 (t, 2H), 7.58 (d, 2H), 7.65 (d, 2H), 8.08 (d, 2H).

### EXAMPLE 2b (method of preparation and isolation of the crystalline salt of AHU-377 with iso-propylamine)

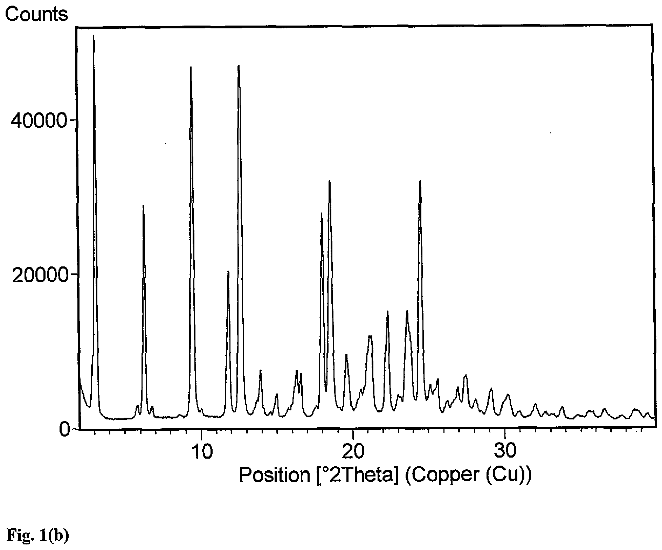

1 g of AHU-377 acid (**1**) was dissolved in 10 ml of ethyl acetate. *Iso*-propylamine was added to the solution in an amount corresponding to 1.1 equivalents of AHU-377. The mixture was agitated. The precipitated crystalline salt of the amine with AHU-377 (**9**) was isolated by filtration, washed with mother liquor and with the solvent used and dried *in vacuo.* The yield of the salt with *iso*-propylamine was 79.6%, the chemical purity according to HPLC was 99.5%. The result of XRPD is presented in Table 8 and in Fig. 1 (b). ¹H NMR (DMSO-D6): 1.06 (d, 3H), 1.10 (d, 6H), 1.13 (t, 3H), 1.38 (m, 1H), 1.76 (m, 1H), 2.22 (s, 3H), 2.24 (m, 1H), 2.68 (m, 2H), 3.14 (m, 1H), 3.91 (m, 1H), 4.00 (q, 2H), 7.27 (d, 2H), 7.35 (t, 1H), 7.46 (t, 2H), 7.58 (d, 2H), 7.66 (d, 2H), 8.03 (d, 2H).

### EXAMPLE 2c (method of preparation and isolation of the crystalline salt of AHU-377 with cyclohexylamine)

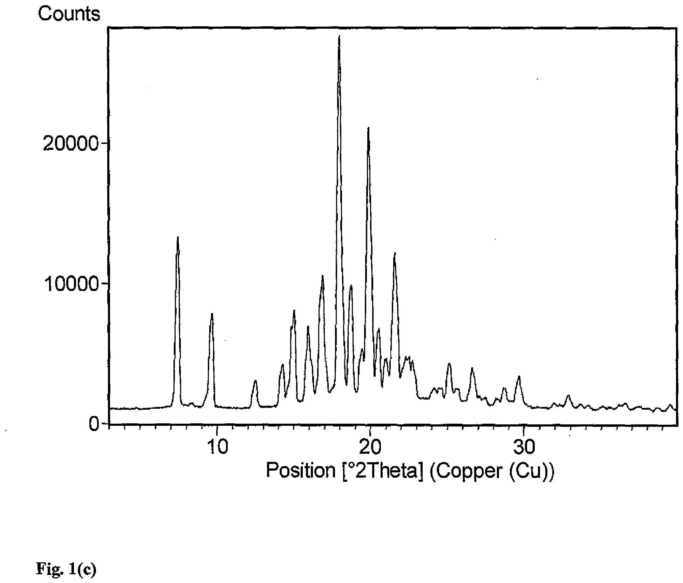

1 g of AHU-377 acid (**1**) was dissolved in 10 ml of ethyl acetate. Cyclohexylamine was added to the solution in an amount corresponding to 1.1 equivalents of AHU-377. The mixture was agitated. The precipitated crystalline salt of the amine with AHU-377 (**9**) was isolated by filtration, washed with mother liquor and with the solvent used and dried *in vacuo.* The yield of the salt with cyclohexylamine was 84.5%, the chemical purity according to HPLC was 99.5%. The result of XRPD is presented in Table 9 and in Fig. 1 (c). ¹H NMR (DMSO-D6): 1.06 (d, 3H), 1.09-1.25 (m, 3H), 1.13 (t, 3H), 1.38 (m, 1H), 1.55 (m, 1H), 1.68 (m, 2H), 1.76 (m, 1H), 1.82 (m, 2H), 2.22 (s, 3H), 2.23 (m, 1H), 2.63-2.74 (m, 3H), 3.91 (m, 1H), 4.00 (q, 2H), 7.27 (d, 2H), 7.35 (t, 1H), 7.46 (t, 2H), 7.58 (d, 2H), 7.66 (d, 2H), 8.04 (d, 2H).

### EXAMPLE 2d (method of preparation and isolation of the crystalline salt of AHU-377 with n-propylamine)

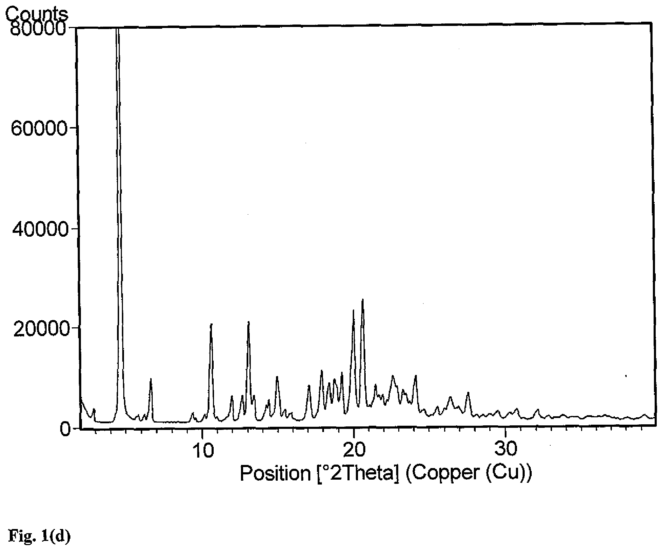

1 g of AHU-377 acid (**1**) was dissolved in 10 ml of ethyl acetate. *N*-propylamine was added to the solution in an amount corresponding to 1.1 equivalents of AHU-377. The mixture was agitated. The precipitated crystalline salt of the amine with AHU-377 (**9**) was isolated by filtration, washed with mother liquor and with the solvent used and dried *in vacuo.* The yield of the salt with *n*-propylamine was 69.8 %, the chemical purity according to HPLC was 99.5 %. The result of XRPD is presented in Table 10 and in Fig. 1 (d). ¹H NMR (DMSO-D6): 0.88 (t, 3H), 1.06 (d, 3 H), 1.13 (t, 3H), 1.38 (m, 1H), 1.49 (m, 2H), 1.76 (m, 1H), 2.22 (s, 3H), 2.24 (m, 1H), 2.63 (t, 2H), 2.68 (m, 2H), 3.91 (m, 1H), 4.00 (q, 2H), 7.27 (d, 2H), 7.35 (t, 1H), 7.46 (t, 2H), 7.58 (d, 2H), 7.66 (d, 2H), 7.99 (d, 2H).

### EXAMPLE 2e (method of preparation and isolation of the crystalline salt of AHU-377 with dicyclohexylamine)

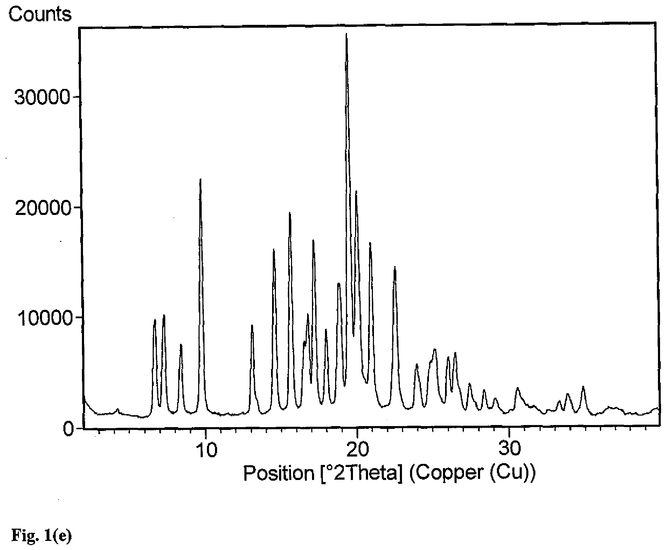

1 g of AHU-377 acid (**1**) was dissolved in 10 ml of ethyl acetate. Dicyclohexylamine was added to the solution in an amount corresponding to 1.1 equivalents of AHU-377. The mixture was agitated. The precipitated crystalline salt of the amine with AHU-377 (**9**) was isolated by filtration, washed with mother liquor and with the solvent used and dried *in vacuo.* The yield of the salt with dicyclohexylamine was 53.6%, the chemical purity according to HPLC was 98.6%. The result of XRPD is presented in Table 11 and in Fig. 1 (e). ¹H NMR (DMSO-D6): 1.04-1.15 (m, 6H), 1.06 (d, 3H), 1.13 (t, 3H), 1.18-1.26 (m, 4H), 1.39 (m, 1H), 1.57 (m, 2H), 1.68 (m, 4H), 1.76 (m, 1H), 1.85 (m, 4H), 2.21-2.32 (m, 4H), 2.64-2.74 (m, 4H), 3.91 (m, 1H), 4.00 (q, 2H), 7.27 (d, 2H), 7.35 (t, 1H), 7.46 (t, 2H), 7.58 (d, 2H), 7.66 (d, 2H), 7.92 (d, 2H).

### EXAMPLE 3 (general method of recrystallization of salts of AHU-377 with amines)

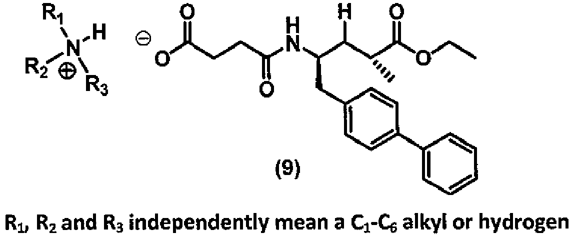

Crude salts of crystalline amines with AHU-377 were dissolved in ethyl acetate or *iso*-propyl acetate in a hot state. The solution was stirred under slow cooling. The precipitated crystalline salt of the amine with AHU-377 (**9**) was isolated by filtration, washed with mother liquor and with the solvent used and dried *in vacuo.* The chemical purity was verified with the use of HPLC, see Table 1. Using an analogous procedure, the crystalline salts with amines were also recrystallized in other organic solvents or in mixtures of more solvents.

**Table 6 - Preparation, isolation and recrystallization of salts of various amines with AHU-377**

| **Amine** | **Precipitated form** | **HPLC (%) after isolation** | **(%) crystallization yield** | **HPLC (%) crystallization** |
|---|---|---|---|---|
| *tert*-butylamine | solid state | 99.4 | 90.3 | 99.9 |
| *iso*-propylamine | solid state | 99.5 | 91.2 | 99.9 |
| cyclohexylamine | solid state | 99.5 | 93.7 | 99.9 |
| *n*-propylamine | solid state | 99.4 | 88.8 | 99.7 |
| dicyclohexylamine | solid state | 98.6 | 72.4 | 99.6 |
| di-*iso*-propylamine | not separated | ------- | ------- | ------- |
| di-*n*-propylamine | not separated | ------- | ------- | ------- |
| di-*iso*-propylethylamine | not separated | ------- | ------- | ------- |
| triethylamine | not separated | ------- | ------- | ------- |
| triethanolamine | solid state | 98.5 | ------- | ------- |
| tris(hydroxxmethyl)aminomethane | solid state | 98.1 | ------- | ------- |
| nicotinamide | not separated | ------- | ------ | ------- |
| L-arginine | not separated | ------- | ------- | ------- |
| diethylamine | not separated | ------- | ------- | ------- |
| *N,N'-*dibenzylethylenediamine | not separated | ------- | ------- | ------- |
| diethanolamine | not separated | ------- | ------- | ------- |

### EXAMPLE 4 (salt of AHU-377 with cyclohexylamine)

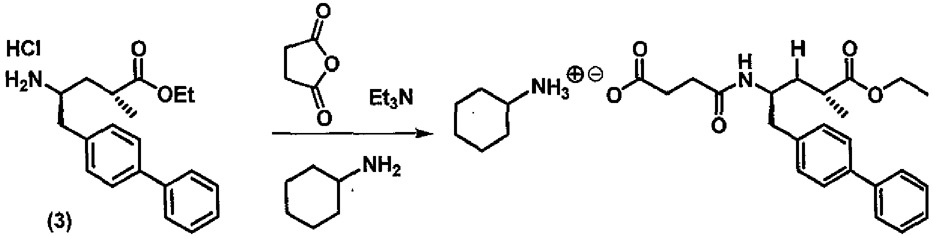

100 ml of ethyl acetate and 6 ml of triethylamine was added to 10 g of the hydrochloride of amine (**3**). The mixture was agitated at the room temperature for approx. 10 minutes. This was followed by addition of 4.3 g of succinic anhydride and agitation of the mixture at the room temperature for 5 hours. Then, 6.5 ml of cyclohexylamine was added and the mixture was agitated at the room temperature. The mixture was inoculated with the crystalline salt of AHU-377 with cyclohexylamine prepared using the method according to Example 2c. The precipitated crystalline salt of cyclohexylamine with AHU-377 was isolated by filtration, washed with mother liquor, the used solvent and dried *in vacuo.* The product yield was 83%, the chemical purity according to HPLC was 99.3%.

### EXAMPLE 5 (amorphous form of the sodium salt of AHU-377)

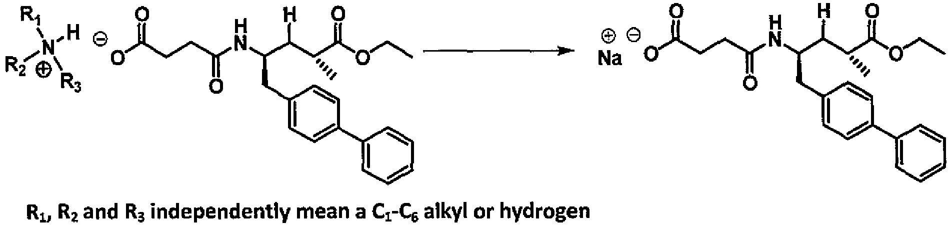

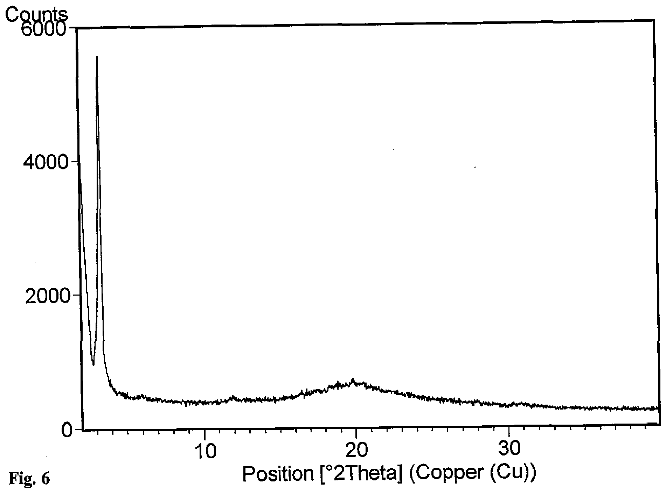

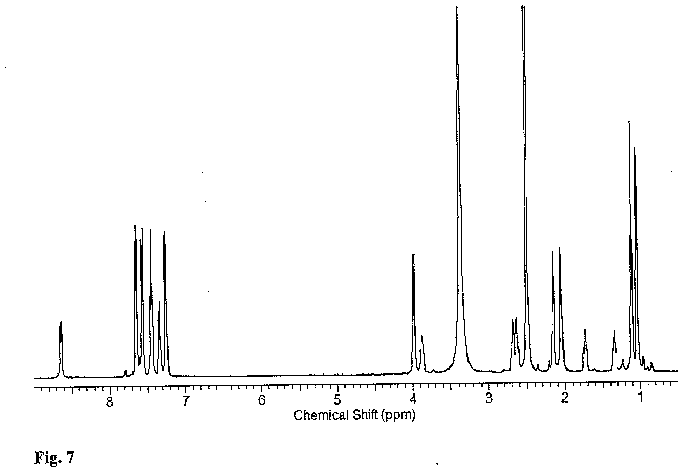

Tetrahydrofuran was added to the crystalline salt of AHU-377 with a primary amine (*tert-*butylamine, *iso*-propylamine, or cyclohexylamine), the suspension was agitated and 1 equivalent of sodium *tert*-butoxide was added. The mixture was stirred at the room temperature until a slightly turbid solution was obtained. Then, it was filtered and heptane was added to the clear filtrate until a turbid solution was produced. This was followed by concentration *in vacuo* and addition of a mixture of heptane and dichloromethane in the constituent ratio of 3:1. The turbid solution was concentrated *in vacuo* and the obtained suspension was agitated at the room temperature. Filtering and vacuum drying followed. The isolated amorphous sodium salt was characterized by means of XRPD (Fig. 6). The product structure was verified with the use of ¹H NMR (Fig. 7).

### EXAMPLE 6 (calcium salt of AHU-377)

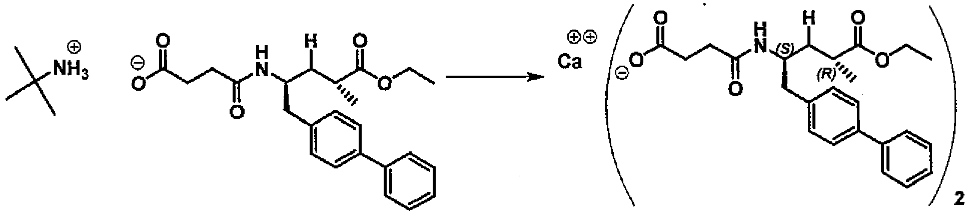

The *tert*-butylammonium salt of AHU-377 (0.5 g) was stirred up in 5 ml of water and calcium chloride dihydrate dissolved in water (1 g in 5 ml of water) was added. The mixture was agitated at 50°C for 3 hours, then it was cooled down to 25°C. The precipitated crystalline product was removed by filtration and dried (0.42 g, 91%, 99.67% HPLC).

### EXAMPLE 7 (calcium salt of AHU-377)

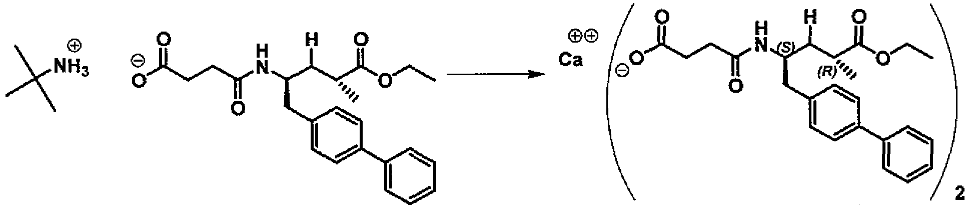

The *tert*-butylammonium salt of AHU-377 (0.5 g) was stirred up in 5 ml of water and calcium acetate dissolved in water (0,85 g in 5 ml of water) was added. The mixture was agitated at 50°C for 3 hours, then it was cooled down to 25°C. The precipitated crystalline product was removed by filtration and dried (0.42 g, 91%, 99.67% HPLC).

### EXAMPLE 8 (potassium salt of AHU-377)

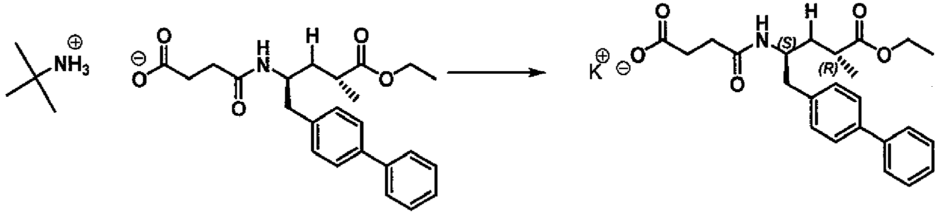

The *tert*-butylammonium salt of AHU-377 (0.5 g) was stirred up in 10 ml of water and 10 ml of ethyl acetate. After acidification of the mixture with hydrochloric acid to pH 1 the organic phase containing the free acid of AHU-377 was separated and the mixture was evaporated in a vacuum evaporator. The evaporation product was dissolved again in 10 ml of methyl ethyl ketone and the potassium salt of 2-ethylhexanoic acid (0.35 g) was added. The mixture was agitated at 50°C for one hour, then it was cooled down to 25°C and the precipitated potassium salt of AHU-377 was removed by filtration and dried. 0.40 g of the salt (87%, 99.65% HPLC) was obtained.

### EXAMPLE 9 (sodium salt of AHU-377)

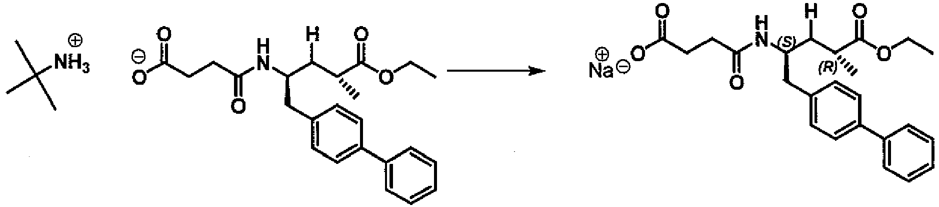

The *tert*-butylammonium salt of AHU-377 (0.5 g) was stirred up in 10 ml of water and 10 ml of ethyl acetate. After acidification of the mixture with hydrochloric acid to pH 1 the organic phase containing the acid was separated and the mixture was evaporated in a vacuum evaporator. The evaporation product was dissolved again in 10 ml of methyl ethyl ketone and the sodium salt of 2-ethylhexanoic acid (0.31 g) was added. The mixture was agitated at 50°C for one hour, then it was cooled down to 25°C and the precipitated sodium salt of AHU-377 was removed by filtration and dried. 0.39 g of the salt (87 %, 99.85% HPLC, melt. point 159 °C - 160°C) was obtained.

### EXAMPLE 10 (sodium salt of AHU-377)

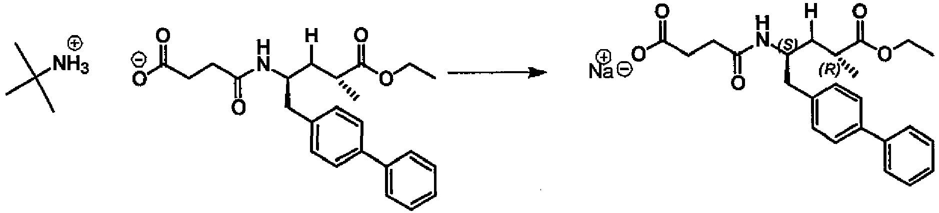

The *tert*-butylammonium salt of AHU-377 (0.5 g) was stirred up in 10 ml of water and 10 ml of ethyl acetate. After acidification of the mixture with hydrochloric acid to pH 1 the organic phase containing the acid was separated and the mixture was evaporated in a vacuum evaporator. The evaporation product was dissolved again in 10 ml of tetrahydrofuran and sodium methoxide (0.2 g) in methanol (1 ml) was added. The mixture was agitated at 50°C for 1 hour, then it was cooled down to 25°C and heptane (15 ml) was added and the precipitated sodium salt of AHU377 was removed by filtration and dried. 0.39 g of the amorphous salt (87%, 99.65% HPLC) was obtained.

### EXAMPLE 11 (semicrystalline calcium salt of AHU-377)

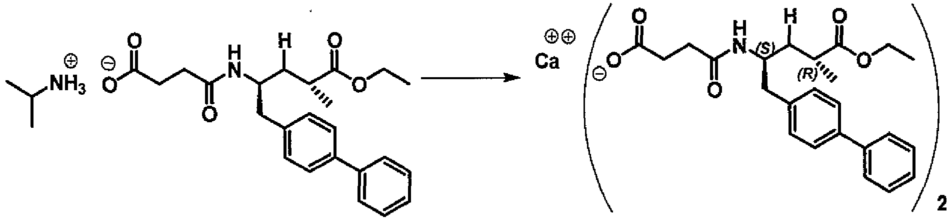

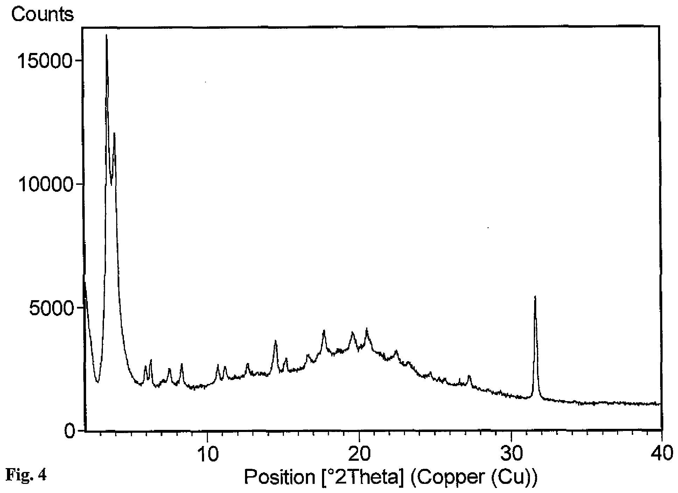

Toluene and 1 equivalent of calcium acetate were added to the crystalline salt of AHU-377 with *iso*-propylamine. The mixture was further agitated, heated up to boiling and a part of the solvent was removed by distillation. Heptane was added to the still warm reaction mixture until a suspension was obtained. This suspension was agitated at the room temperature. Finally, filtration and vacuum drying of the semicrystalline calcium salt was done (yield 90%, XPRD pattern see Fig. 4).

### EXAMPLE 12 (calcium salt of AHU-377)

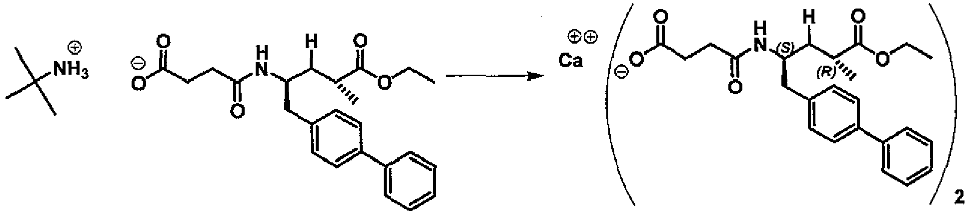

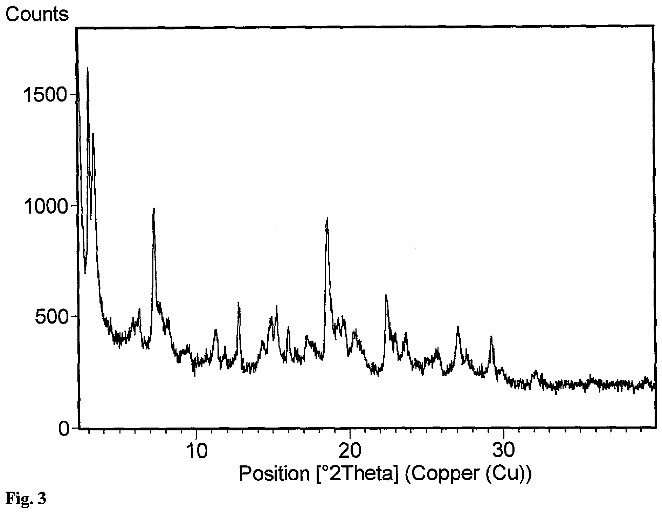

The *tert*-butylammonium salt of AHU-377 (0.1 g) was stirred up in 2 ml of water and 2 ml of ethyl acetate. After acidification of the mixture with hydrochloric acid to pH 1 the organic phase containing the acid was separated and the mixture was evaporated in a vacuum evaporator. The evaporation product was dissolved in 2 ml of acetone and 0.243 ml of a suspension of calcium hydroxide was added that had been prepared by suspending (0.074 g) in water (1 ml). The mixture was homogenized in an ultrasonic bath and then it was left to evaporate in a vacuum drier at the room temperature. 0.113 g of the crystalline salt (96%, 98% HPLC) was obtained. The crystalline calcium salt was characterized by means of XRPD (Fig. 3).

### EXAMPLE 13 (tris(hydroxmethyl)aminomethane salt of AHU-377) (Reference Example)

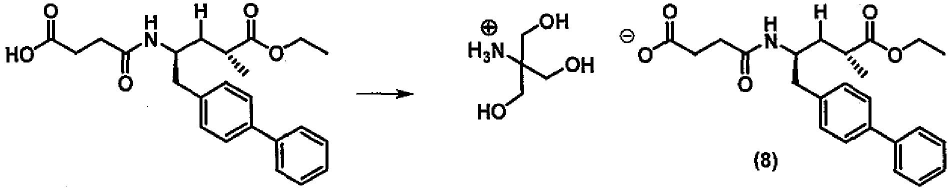

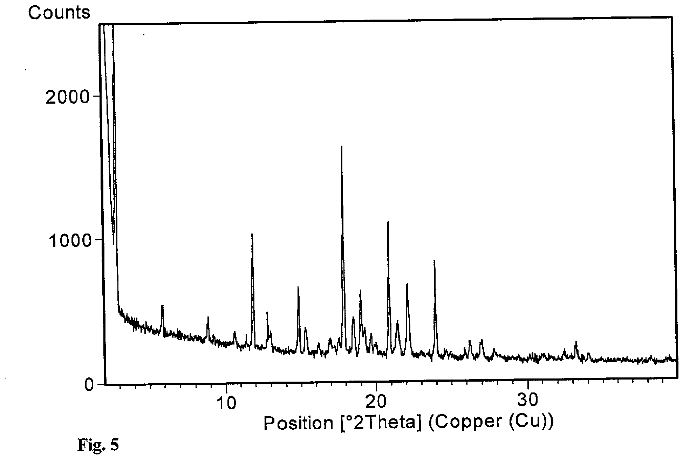

0.085 g of AHU-377 acid (**1**) was dissolved in ethyl acetate (2 ml) and 0.294 ml of a solution of tris(hydroxmethyl)aminomethane (0.100 g in 1 ml of water) was added. The mixture was homogenized in an ultrasonic bath and then it was evaporated in a vacuum drier at the room temperature. The crystalline salt (HPLC 98.1%) was characterized by means of XRPD (Fig. 5). The salt of AHU-377 with triethanolamine was prepared in an analogous way (HPLC 98.5%).

### Example 14 - salt of sacubitril with cyclohexyl amine

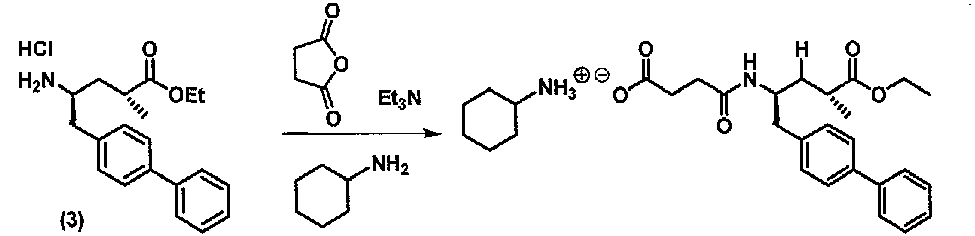

100 ml of ethyl acetate and 6 ml of triethylamine was added to 10 g of the amine hydrochloride (**3**). The mixture was agitated at the room temperature for approx. 10 minutes. This was followed by addition of 4.3 g of succinanhydride and agitation of the mixture at the room temperature for 5 hours. Then, 6.5 ml of cyclohexylamine was added and the mixture was agitated at the room temperature. The mixture was inoculated with the crystalline salt of sacubitril with cyclohexylamine prepared in the previous experiments. The precipitated crystalline salt of cyclohexylamine with sacubitril was isolated by filtration, washed with mother liquor, with the solvent used, and dried *in vacuo.*

### Example 15 - calcium salt of sacubitril

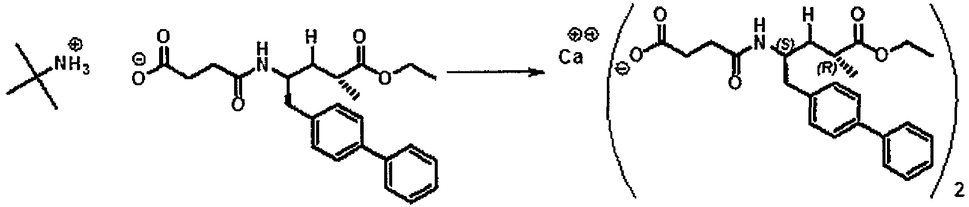

210 ml of water was added to 21 g of the cyclohexyl ammonium salt of sacubitril in a 500ml three-neck flask. The resulting solution was tempered at 30°C in a bath under stirring. Further, a solution of calcium chloride that was prepared by dissolution of 6.05 g of CaCl₂·2H₂O in 30 ml of water, was added dropwise under intensive stirring. The resulting suspension was left to be stirred at 30°C for 30 min. Then, the solid fraction was removed by filtration. The filtration cake was washed with 80 ml of water in two fractions and it was left on frit for approx. 1h. The wet filtration cake was dried in a vacuum drier at the pressure of 15 kPa at the room temperature for 12 h and then at 45°C for 24 hours.

### Example 16 - Preparation of crystalline form I of the calcium salt of sacubitril (Reference Example)

The charge of the calcium salt of sacubitril of 104.0 mg was dissolved in 0.3 ml of ethanol (98 %) in a glass vial at 78°C while being stirred in a magnetic agitator. The resulting solution was left to cool down freely and to evaporate in an extraction cupboard for 48 hours. X-ray powder pattern in Fig. 8. Melting point 86°C according to DSC.

### Example 17 - Preparation of crystalline form II of the calcium salt of sacubitril (Reference Example)

The charge of the calcium salt of sacubitril of 99.4 mg was dissolved in 0.4 ml of a dioxane:water mixture (1:1, *V*:*V*) in a glass vial at 100°C while being stirred in a magnetic agitator. The resulting solution was evaporated in a vacuum drier at the temperature of 25°C and the pressure of 20 kPa. X-ray powder pattern in Fig. 9. Melting point 68°C according to DSC.

### Example 18 - Preparation of semicrystalline form III of the calcium salt of sacubitril (Reference Example)

The charge of the calcium salt of sacubitril of 103.6 mg was dissolved in 0.2 ml of tetrahydrofuran in a glass vial at 66°C while being stirred in a magnetic agitator. The resulting solution was evaporated in a vacuum drier at the temperature of 25°C and the pressure of 20 kPa. X-ray powder pattern in Fig. 10. Melting point 124°C according to DSC.

### Example 19 - Preparation of semicrystalline form IV of the calcium salt of sacubitril (Reference Example)

The charge of the calcium salt of sacubitril of 104.5 mg was dissolved in 0.2 ml of chloroform at 58°C while being stirred in a magnetic agitator. The resulting solution was quickly cooled down to -20°C by being inserted into an ice bath containing NaCl. The solid fraction was separated from the solution by filtration. The filtration cake was left to freely dry in an extraction cupboard for 48 hours. X-ray powder pattern in Fig. 11. Melting point 173°C according to DSC.

### ANALYTICAL METHODS AND DATA (A, B, C):

### A High-performance liquid chromatography (HPLC)

The HPLC chromatograms were measured using a UHPLC system Agilent 1290 Infinity LC. For the analyses an ACQUITY CSH Phenyl-Hexyl; 100 mm x 3.0 mm I. D.; 1.7 µm column was used. As the mobile phase a mixture of acetonitrile (70 %) and HClO₄ (30 %, 1 ml of HClO₄ per 11 of water) was used. The measurements were carried out in a gradient mode with the mobile phase flow of 0.5 ml/min at 40°C in the column.

### B X-ray powder diffraction (XRPD)

The XRPD diffractograms of the crystalline salts of AHU-377 with amines that were prepared according to Example 3 were measured with the use of an X'PERT PRO MPD PANalytical diffractometer under the following experimental conditions:
Radiation: CuKα (λ = 1.54178 Å)
Excitation voltage: 45 kV
Anode current: 40 mA
Measured range: 2 - 40° 2θ
Increment: 0,01 2θ

The values of the measured characteristic diffraction angles 2θ, interplanar spacings d and relative signal intensities are presented in Tables 2-6.

**Table 7 - Values of characteristic diffraction angles 2θ, interplanar spacings d and relative signal intensities in the XRPD patterns of the crystalline salt of AHU-377 with tert-butylamine**

| **Pos. [°2Th.]** | **Interplanar spacing d (Å)** | **Rel. int. (%)** |
|---|---|---|
| 4.03 | 21.906 | 20.1 |
| 8.00 | 11.047 | 100.0 |
| 9.82 | 9.002 | 26.1 |
| 12.31 | 7.187 | 6.4 |
| 13.02 | 6.793 | 10.3 |
| 15.11 | 5.858 | 18.0 |
| 15.64 | 5.661 | 9.9 |
| 16.12 | 5.495 | 24.5 |
| 16.71 | 5.302 | 11.3 |
| 17.77 | 4.986 | 84.0 |
| 18.99 | 4.671 | 16.3 |
| 19.56 | 4.534 | 25.4 |
| 20.20 | 4.393 | 57.8 |
| 21.59 | 4.114 | 15.9 |
| 23.03 | 3.859 | 19.4 |
| 24.50 | 3.631 | 8.4 |
| 26.18 | 3.402 | 7.5 |
| 27.36 | 3.257 | 9.8 |
| 28.61 | 3.117 | 5.6 |

**Table 8 - Values of characteristic diffraction angles 2θ, interplanar spacings d and relative signal intensities in the XRPD patterns of the crystalline salt of AHU-377 with iso-propylamine**

| **Pos. [°2Th.]** | **Interplanar spacing d (Å)** | **Rel. int. (%)** |
|---|---|---|
| 3.14 | 28.109 | 100.0 |
| 6.28 | 14.067 | 55.8 |
| 9.46 | 9.346 | 89.7 |
| 11.79 | 7.498 | 38.1 |
| 12.62 | 7.007 | 99.1 |
| 13.90 | 6.367 | 11.4 |
| 14.93 | 5.930 | 6.0 |
| 16.29 | 5.437 | 11.3 |
| 16.56 | 5.349 | 11.0 |
| 18.03 | 4.916 | 52.5 |
| 18.55 | 4.780 | 61.9 |
| 19.55 | 4.537 | 13.8 |
| 21.14 | 4.199 | 17.7 |
| 22.34 | 3.976 | 27.1 |
| 23.59 | 3.768 | 26.3 |
| 24.57 | 3.621 | 60.1 |
| 25.56 | 3.483 | 10.6 |
| 27.33 | 3.261 | 8.0 |
| 29.02 | 3.075 | 6.9 |
| 30.16 | 2.961 | 5.1 |

**Table 9 - Values of characteristic diffraction angles 2θ, interplanar spacings d and relative signal intensities in the XRPD patterns of the crystalline salt of AHU-377 with cyclohexylamine**

| **Pos. [°2Th.]** | **Interplanar spacing d (Å)** | **Rel. int. (%)** |
|---|---|---|
| 7.44 | 11.879 | 47.6 |
| 9.70 | 9.111 | 18.0 |
| 12.45 | 7.106 | 5.9 |
| 14.27 | 6.203 | 10.9 |
| 15.03 | 5.890 | 25.0 |
| 15.95 | 5.554 | 20.3 |
| 16.86 | 5.254 | 34.6 |
| 18.01 | 4.921 | 100.0 |
| 18.68 | 4.747 | 25.5 |
| 19.92 | 4.454 | 71.8 |
| 20.59 | 4.311 | 15.6 |
| 20.99 | 4.228 | 12.0 |
| 21.59 | 4.112 | 40.8 |
| 22.27 | 3.989 | 11.3 |
| 22.77 | 3.903 | 10.5 |
| 25.16 | 3.537 | 10.5 |
| 26.61 | 3.347 | 9.6 |
| 28.68 | 3.110 | 5.2 |
| 29.64 | 3.011 | 7.7 |

**Table 10 - Values of characteristic diffraction angles 2θ, interplanar spacings d and relative signal intensities in the XRPD patterns of the crystalline salt of AHU-377 with n-propylamine**

| **Pos. [°2Th.]** | **Interplanar spacing d (Å)** | **Rel. int. (%)** |
|---|---|---|
| 4.76 | 18.557 | 100.0 |
| 6.68 | 13.218 | 4.4 |
| 10.67 | 8.287 | 9.6 |
| 11.91 | 7.423 | 3.5 |
| 12.67 | 6.984 | 2.5 |
| 13.08 | 6.763 | 12.4 |
| 14.96 | 5.917 | 5.7 |
| 17.08 | 5.188 | 3.6 |
| 17.89 | 4.955 | 5.3 |
| 18.43 | 4.811 | 3.4 |
| 19.23 | 4.613 | 5.0 |
| 20.05 | 4.424 | 11.4 |
| 20.68 | 4.291 | 13.4 |
| 21.47 | 4.136 | 3.3 |
| 22.63 | 3.926 | 4.9 |
| 23.26 | 3.821 | 2.7 |
| 24.15 | 3.683 | 4.6 |
| 26.38 | 3.376 | 2.4 |
| 27.53 | 3.237 | 3.2 |
| 30.68 | 2.912 | 1.2 |

**Table 11 - Values of characteristic diffraction angles 2θ, interplanar spacings d and relative signal intensities in the XRPD patterns of the crystalline salt of AHU-377 with dicyclohexylamine**

| **Pos. [°2Th.]** | **Interplanar spacing d (Å)** | **Rel. int. (%)** |
|---|---|---|
| 6.70 | 13.177 | 27.1 |
| 7.30 | 12.106 | 27.4 |
| 8.40 | 10.517 | 19.3 |
| 9.77 | 9.048 | 63.0 |
| 13.11 | 6.749 | 24.8 |
| 14.60 | 6.064 | 46.6 |
| 15.65 | 5.659 | 55.9 |
| 16.80 | 5.275 | 23.8 |
| 17.21 | 5.148 | 47.5 |
| 17.98 | 4.930 | 22.3 |
| 18.83 | 4.709 | 30.3 |
| 19.55 | 4.538 | 100.0 |
| 20.09 | 4.417 | 59.0 |
| 20.98 | 4.230 | 45.2 |
| 22.54 | 3.941 | 39.0 |
| 23.93 | 3.715 | 12.5 |
| 25.13 | 3.541 | 17.2 |
| 26.02 | 3.421 | 15.4 |
| 26.53 | 3.357 | 13.5 |
| 27.41 | 3.252 | 8.3 |
| 28.36 | 3.145 | 6.7 |
| 30.57 | 2.922 | 5.9 |
| 33.86 | 2.645 | 5.6 |
| 34.83 | 2.574 | 7.6 |

**Table 12 - Values of characteristic diffraction angles 2θ, interplanar spacings d and relative signal intensities in the XRPD patterns of the crystalline calcium salt of AHU-377.**

| **Pos. [°2Th.]** | **Interplanar spacing d (Å)** | **Rel. int. (%)** |
|---|---|---|
| 3.11 | 28.430 | 100.0 |
| 3.41 | 25.874 | 71.4 |
| 6.31 | 14.007 | 15.2 |
| 7.31 | 12.080 | 66.7 |
| 11.36 | 7.785 | 13.8 |
| 12.80 | 6.909 | 24.7 |
| 14.92 | 5.932 | 18.9 |
| 15.24 | 5.808 | 22.3 |
| 16.06 | 5.514 | 15.3 |
| 18.61 | 4.763 | 60.6 |
| 20.34 | 4.363 | 10.4 |
| 22.51 | 3.947 | 32.4 |
| 23.72 | 3.748 | 12.2 |
| 27.05 | 3.293 | 19.8 |
| 29.22 | 3.053 | 17.3 |

**Table 13 - Values of characteristic diffraction angles 2θ, interplanar spacings d and relative signal intensities in the XRPD patterns of the semicrystalline calcium salt of AHU-377.**

| **Pos. [°2Th.]** | **Interplanar spacing d (Å)** | **Rel. int. (%)** |
|---|---|---|
| 3.58 | 24.648 | 100.0 |
| 4.06 | 21.772 | 71.7 |
| 6.01 | 14.702 | 5.8 |
| 6.35 | 13.907 | 7.9 |
| 7.57 | 11.675 | 5.4 |
| 8.37 | 10.559 | 7.0 |
| 10.74 | 8.233 | 5.1 |
| 11.22 | 7.878 | 4.3 |
| 12.72 | 6.954 | 3.8 |
| 14.54 | 6.088 | 9.0 |
| 15.21 | 5.821 | 3.9 |
| 16.64 | 5.324 | 3.5 |
| 17.74 | 4.995 | 10.7 |
| 19.63 | 4.520 | 9.5 |
| 20.54 | 4.321 | 11.3 |
| 22.46 | 3.956 | 5.2 |
| 23.32 | 3.811 | 3.0 |
| 27.30 | 3.265 | 3.6 |

**Table 14 - Values of characteristic diffraction angles 2θ, interplanar spacings d and relative signal intensities in the XRPD patterns of the crystalline tris(hydroxymethyl)amino-methane salt of AHU-377 (8).**

| **Pos. [°2Th.]** | **Interplanar spacing d (Å)** | **Rel. int. (%)** |
|---|---|---|
| 2.91 | 30.331 | 100.0 |
| 5.90 | 14.961 | 7.7 |
| 8.89 | 9.938 | 6.6 |
| 10.63 | 8.318 | 3.8 |
| 11.89 | 7.440 | 29.1 |
| 13.03 | 6.787 | 4.2 |
| 14.89 | 5.945 | 14.5 |
| 15.27 | 5.798 | 6.0 |
| 17.91 | 4.950 | 32.8 |
| 18.46 | 4.803 | 7.2 |
| 18.99 | 4.670 | 15.0 |
| 19.66 | 4.513 | 4.8 |
| 20.91 | 4.246 | 31.0 |
| 21.45 | 4.139 | 7.5 |
| 22.08 | 4.023 | 17.9 |
| 23.95 | 3.713 | 24.6 |
| 26.22 | 3.397 | 4.1 |
| 26.95 | 3.306 | 3.8 |
| 33.20 | 2.697 | 4.7 |

### C ¹H NMR

The ¹H NMR spectra were measured using a Bruker Avance 500 spectrometer with the measuring frequency of 500.131 MHz. The spectra were measured for solutions in DMSO-D6, the chemical shifts were related to the internal standard of TMS δ = 0 ppm.

### Differential Scanning Calorimetry (DSC)

The records of the solid calcium salts of sacubitril were measured with the Discovery DSC device made by TA Instruments. The sample charge in a standard A1 pot (40 µL) was between 4-5 and 5 mg and the heating rate was 5°C/min. The temperature program that was used consists of 1 min of stabilization at the temperature of 0°C and then of heating up to 250°C at the heating rate of 5°C/min (Amplitude = 0.8°C and Period = 60 s). As the carrier gas 5.0 N₂ was used at the flow of 50 ml/min.

## Claims

1. A method for the preparation of crystalline salts of AHU-377 with amines of the general formula **9**, wherein R₁, R₂, R₃ independently mean a C1-C6 alkyl or hydrogen, **characterized in that** the AHU-377 acid described by formula **1** is reacted with the respective amine in at least one organic solvent or in a mixture thereof, followed by isolation of the crystalline salt of the amine with AHU-377 of formula **9** 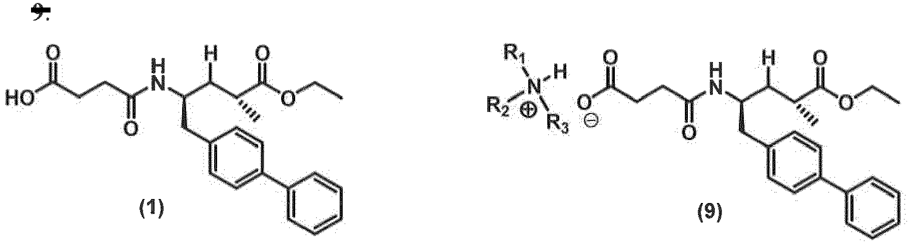 and wherein the amine used is selected from the group comprising *tert*-butylamine, *iso*-propylamine, cyclohexylamine, *n*-propylamine and dicyclohexylamine.

2. The method according to claim 1, **characterized in that** the preparation of the crystalline salts of AHU-377 with amines comprises the following steps:
(a) reaction of amines with AHU-377 carboxylic acid of formula **1** in a solution of at least one organic solvent, or in a mixture thereof,
(b) subsequent isolation of crystalline salts of formula **9**, wherein the amine contained therein is selected from the group comprising *tert*-butylamine, *iso*-propylamine, cyclohexylamine, *n*-propylamine and dicyclohexylamine, and
(c) recrystallization.

3. The method according to claims 1 or 2, **characterized in that** at least one organic solvent is used, selected from the group comprising esters of C1-C5 organic acids with C1-C6 alcohols, C1-C6 alcohols, C3-C6 ketones, C4-C6 ethers, C1-C6 chlorinated hydrocarbons, liquid aromatic hydrocarbons, C1-C7 alkanes, C1-C7 cycloalkanes, or mixtures thereof, preferably ethyl acetate, *iso*-propyl acetate, methanol, ethanol, *iso*-propyl alcohol, acetone, methyl ethyl ketone, diethyl ether, tetrahydrofuran, 2-methyltetrahydrofuran, *tert-*butyl methyl ether, dichloromethane, toluene, xylenes, hexane, heptane, cyclohexane, or mixtures thereof.

4. A method for the recrystallization of the crystalline salts of AHU-377 with amines of the general formula **9** according to claims 1 or 2, **characterized in that** at least one organic solvent is used, selected from the group comprising esters of C1-C5 organic acids with C1-C6 alcohols, C1-C6 alcohols, C3-C6 ketones, C4-C6 ethers, C1-C6 chlorinated hydrocarbons, liquid aromatic hydrocarbons, C1-C7 alkanes, C1-C7 cycloalkanes, or mixtures thereof, preferably ethyl acetate, *iso*-propyl acetate, methanol, ethanol, *iso*-propyl alcohol, acetone, methyl ethyl ketone, diethyl ether, tetrahydrofuran, 2-methyltetrahydrofuran, *tert*-butyl methyl ether, dichloromethane, toluene, xylenes, hexane, heptane, cyclohexane, or mixtures thereof.

5. Crystalline salts of AHU-377 with amines of formula **9** as defined by claim 1, wherein this salt comprises a primary amine selected from the group comprising cyclohexylamine, *iso*-propylamine and *tert*-butylamine, *n*-propylamine and dicyclohexylamine.

6. The crystalline salt of AHU-377 according to claim 5 with cyclohexylamine, **characterized by** the following reflections in XRPD: 7.4, 15.0, 18.0, 19.9, 21.6 (° ± 0.2° 2θ).

7. The crystalline salt of AHU-377 according to claim 5 with *iso*-propylamine, **characterized by** the following reflections in XRPD: 3.1, 9.5, 12.6, 18.5, 24.6 (° ± 0.2° 2θ).

8. The crystalline salt of AHU-377 according to claim 5 with *tert*-butylamine, **characterized by** the following reflections in XRPD: 8.0, 9.8, 16.1, 17.8, 20.2 (° ± 0.2° 2θ).

9. A method for the preparation and isolation of the crystalline salts of AHU-377 with amines defined in claims 5, 6, 7 or 8, **characterized in that** it comprises the following steps:
(a) Dissolving the AHU-377 acid in an organic solvent,
(b) adding the amine to the solution of step (a),
(c) agitating the solution of step (b),
(d) filtering the precipitated solid form and
(e) drying the crystalline salt.

10. The method according to claim 9, **characterized in that** at least one organic solvent is used, selected from the group comprising esters of C1-C5 organic acids with C1-C6 alcohols, C1-C6 alcohols, C3-C6 ketones, C4-C6 ethers, C1-C6 chlorinated hydrocarbons, liquid aromatic hydrocarbons, C1-C7 alkanes, C1-C7 cycloalkanes, or mixtures thereof, preferably ethyl acetate, *iso*-propyl acetate, methanol, ethanol, *iso*-propyl alcohol, acetone, methyl ethyl ketone, diethyl ether, tetrahydrofuran, 2-methyltetrahydrofuran, tert-butyl methyl ether, dichloromethane, toluene, xylenes, hexane, heptane, cyclohexane, or mixtures thereof.

11. The method according to claims 9 or 10, which comprises the following steps:
(a) dissolving the AHU-377 acid in ethyl acetate or *iso*-propyl acetate,
(b) adding 0.9 to 1.2 equivalents of an amine selected from the group comprising cyclohexylamine, *iso*-propylamine or *tert*-butylamine to the solution of step (a),
(c) agitating the solution of step (b),
(d) filtering the precipitated solid form and
(e) drying the crystalline salt *in vacuo.*

12. A method of removing chemical impurities from the crystalline salts of AHU-377 with amines obtained according to claims 9, 10 or 11, which is **characterized by** recrystallization of crude salts from an organic solvent, preferably from ethyl acetate or *iso*-propylacetate.

13. A method for the preparation of pharmaceutically acceptable solid forms of the substances of formula **10**, wherein M stands for an alkali metal, and **11**, wherein M stands for an alkaline earth metal, derived from the substance AHU-377 of formula **1**, **characterized in that** salts of AHU-377 with amines obtained according to any of claims 1-4 and 9-11 are reacted with the respective inorganic or organic salt, preferably an alcoholate or hydroxide of a metal from the group of alkali metals or alkaline earth metals.

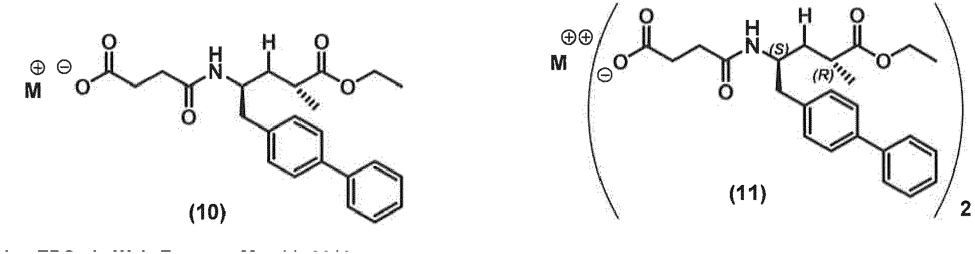

14. The method according to claim 13, **characterized in that** the pharmaceutically acceptable form is the sodium salt of AHU-377 of formula **10**, wherein M stands for sodium, or the calcium salt of AHU-377 of formula **11**, wherein M stands for calcium.

15. The method according to claims 13 or 14, **characterized in that** the crystalline salts of AHU-377 with amines selected from the group of *tert*-butylamine, *iso*-propylamine, cyclohexylamine are chemically converted to the sodium salt of formula **10**, wherein M stands for sodium, or to the calcium salt of formula **11**, wherein M stands for calcium.

16. A method for the preparation of the amorphous sodium salt of AHU-377 of formula **10**, wherein M stands for sodium, according to claims 13, 14 or 15, **characterized in that** the crystalline salts of AHU-377 with amines of formula **9** are treated with an agent selected from the group of inorganic or organic sodium salts, preferably sodium alcoholates, or sodium hydroxide.

17. The method according to claims 13 to 16, **characterized in that** a source sodium compound is used, which is sodium methanolate, sodium ethanolate, sodium *iso-*propoxide, or sodium *tert*-butylate.

18. A method for the preparation of the crystalline or semicrystalline calcium salt of AHU-377 of formula **11**, wherein M stands for calcium, according to claims 13 to 17, **characterized in that** the crystalline salts of AHU-377 with amines of formula **9** are treated with an agent selected from the group of inorganic or organic calcium salts, preferably calcium alcoholates, or calcium hydroxide.

19. The method according to claims 13 to 18, **characterized in that** a source calcium compound is used, which is calcium chloride, calcium carbonate, or calcium acetate.

## Patentansprüche

1. Verfahren zur Herstellung von kristallinen Salzen von AHU-377 mit Aminen der allgemeinen Formel 9, worin R₁, R₂, R₃ unabhängig voneinander ein C1-C6-Alkyl oder Wasserstoff bedeuten, **dadurch gekennzeichnet, dass** die durch Formel 1 beschriebene AHU-377-Säure mit dem jeweiligen Amin in mindestens einem organischen Lösungsmittel oder in einer Mischung davon umgesetzt wird, gefolgt von der Isolierung des kristallinen Salzes des Amins mit AHU-377 der Formel 9, 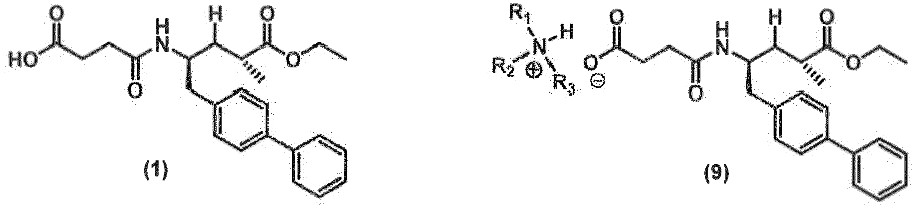 wobei das verwendete Amin aus der Gruppe ausgewählt ist, die tert-Butylamin, *iso*-Propylamin, Cyclohexylamin, n-Propylamin und Dicyclohexylamin umfasst.

2. Verfahren nach Anspruch 1, **dadurch gekennzeichnet, dass** die Herstellung der kristallinen Salze von AHU-377 mit Aminen die folgenden Schritte umfasst:
(a) Umsetzung von Aminen mit AHU-377-Carbonsäure der Formel 1 in einer Lösung mit mindestens einem organischen Lösungsmittel oder in einer Mischung davon,
(b) anschließende Isolierung kristalliner Salze der Formel 9, wobei das darin enthaltene Amin ausgewählt ist aus der Gruppe, die tert-Butylamin, *iso*-Propyl-amin, Cyclohexylamin, n-Propylamin und Dicyclohexylamin umfasst, und
(c) Umkristallisation.

3. Verfahren nach Anspruch 1 oder 2, **dadurch gekennzeichnet, dass** mindestens ein organisches Lösungsmittel verwendet wird, welches ausgewählt ist aus der Gruppe, die Ester von organischen C1-C5-Säuren mit C1-C6-Alkoholen, C1-C6-Alkohole, C3-C6-Ketone, C4-C6-Ether, C1-C6-Chlorkohlenwasserstoffe, flüssige aromatische Kohlenwasserstoffe, C1-C7-Alkane, C1-C7-Cycloalkane oder Mischungen davon, vorzugsweise Ethylacetat, *iso*-Propylacetat, Methanol, Ethanol, iso-Propylalkohol, Aceton, Methylethylketon, Diethylether, Tetrahydrofuran, 2-Methyltetrahydrofuran, tert-Butylmethylether, Dichlormethan, Toluol, Xylole, Hexan, Heptan, Cyclohexan oder Mischungen davon, umfasst.

4. Verfahren zur Umkristallisation der kristallinen Salze von AHU-377 mit Aminen der allgemeinen Formel 9 nach Anspruch 1 oder 2, **dadurch gekennzeichnet, dass** mindestens ein organisches Lösungsmittel verwendet wird, welches ausgewählt ist aus der Gruppe, die Ester von organischen C1-C5-Säuren mit C1-C6-Alkoholen, C1-C6-Alkohole, C3-C6-Ketone, C4-C6-Ether, C1-C6-Chlorkohlenwasserstoffe, flüssige aromatische Kohlenwasserstoffe, C1-C7-Alkane, C1-C7-Cycloalkane oder Gemische davon, vorzugsweise Ethylacetat, *iso*-Propylacetat, Methanol, Ethanol, *iso*-Propylalkohol, Aceton, Methylethylketon, Diethylether, Tetrahydrofuran, 2-Methyltetrahydrofuran, tert-Butylmethylether, Dichlormethan, Toluol, Xylole, Hexan, Heptan, Cyclohexan oder Mischungen davon, umfasst.

5. Kristalline Salze von AHU-377 mit Aminen der Formel 9 nach Anspruch 1, wobei dieses Salz ein primäres Amin umfasst, welches aus der Gruppe ausgewählt ist, die Cyclohexylamin, *iso*-Propylamin und tert-Butylamin, n-Propylamin und Dicyclohexylamin umfasst.

6. Kristallines Salz von AHU-377 nach Anspruch 5 mit Cyclohexylamin, **gekennzeichnet durch** die folgenden Reflexionen im XRPD: 7,4, 15,0, 18,0, 19,9, 21,6 (° ±0,2° 2θ).

7. Kristallines Salz von AHU-377 nach Anspruch 5 mit iso-Propylamin, **gekennzeichnet durch** die folgenden Reflexionen im XRPD: 3,1, 9,5, 12,6, 18,5, 24,6 (° ±0,2° 2θ).

8. Kristallines Salz von AHU-377 nach Anspruch 5 mit *tert-*Butylamin, **gekennzeichnet durch** die folgenden Reflexionen im XRPD: 8,0, 9,8, 16,1, 17,8, 20,2 (° ±0,2° 2θ).

9. Verfahren zur Herstellung und Isolierung der kristallinen Salze von AHU-377 mit Aminen gemäß den Ansprüchen 5, 6, 7 oder 8, **dadurch gekennzeichnet, dass** es die folgenden Schritte umfasst:
(a) Lösen der AHU-377-Säure in einem organischen Lösungsmittel,
(b) Zugeben des Amins zu der Lösung von Schritt (a),
(c) Rühren oder Bewegen der Lösung von Schritt (b),
(d) Filtrieren der ausgefallenen festen Form und
(e) Trocknen des kristallinen Salzes.

10. Verfahren nach Anspruch 9, **dadurch gekennzeichnet, dass** mindestens ein organisches Lösungsmittel verwendet wird, welches ausgewählt ist aus der Gruppe, welche Ester von organischen C1-C5-Säuren mit C1-C6-Alkoholen, C1-C6-Alkohole, C3-C6-Ketone, C4-C6-Ether, C1-C6-Chlorkohlenwasserstoffe, flüssige aromatische Kohlenwasserstoffe, C1-C7-Alkane, C1-C7-Cycloalkane oder Mischungen davon, vorzugsweise Ethylacetat, *iso*-Propylacetat, Methanol, Ethanol, *iso*-Propylalkohol, Aceton, Methylethylketon, Diethylether, Tetrahydrofuran, 2-Methyltetrahydrofuran, tert-Butylmethylether, Dichlormethan, Toluol, Xylole, Hexan, Heptan, Cyclohexan oder Mischungen davon, umfasst.

11. Verfahren nach Anspruch 9 oder 10, welches die folgenden Schritte umfasst:
(a) Lösen der AHU-377-Säure in Ethylacetat oder iso-Propylacetat,
(b) Zugeben von 0,9 bis 1,2 Äquivalenten eines Amins, ausgewählt aus der Gruppe, die Cyclohexylamin, iso-Propylamin oder *tert*-Butylamin umfasst, zu der Lösung aus Schritt (a),
(c) Rühren oder Bewegen der Lösung aus Schritt (b),
(d) Filtrieren der ausgefallenen festen Form und
(e) Trocknen des kristallinen Salzes im Vakuum.

12. Verfahren zum Entfernen chemischer Verunreinigungen aus den gemäß den Ansprüchen 9, 10 oder 11 erhaltenen kristallinen Salzen von AHU-377 mit Aminen, **gekennzeichnet durch** Umkristallisation von Rohsalzen aus einem organischen Lösungsmittel, vorzugsweise aus Ethylacetat oder *iso*-Propylacetat.

13. Verfahren zur Herstellung von pharmazeutisch akzeptablen festen Formen der Substanzen der Formel 10, worin M für ein Alkalimetall steht, und 11, worin M für ein Erdalkalimetall steht, als Derivate der Substanz AHU-377 der Formel 1, **dadurch gekennzeichnet, dass** Salze von AHU-377 mit Aminen, welche nach einem der Ansprüche 1-4 und 9-11 erhalten wurden, mit dem jeweiligen anorganischen oder organischen Salz, vorzugsweise einem Alkoholat oder Hydroxid eines Metalls aus der Gruppe der Alkalimetalle oder Erdalkalimetalle, umgesetzt werden.

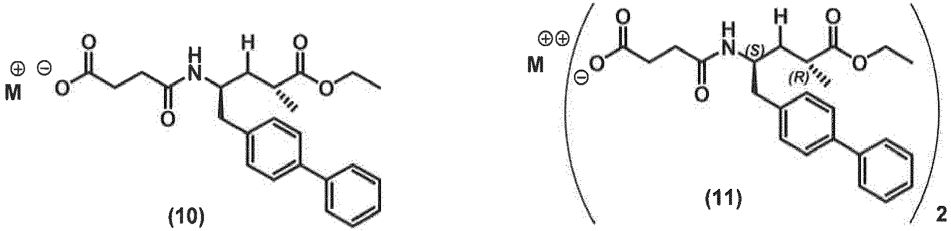

14. Verfahren nach Anspruch 13, **dadurch gekennzeichnet, dass** die pharmazeutisch akzeptable Form das Natriumsalz von AHU-377 der Formel 10, worin M für Natrium steht, oder das Calciumsalz von AHU-377 der Formel 11, worin M für Kalzium steht, ist.

15. Verfahren nach den Ansprüchen 13 oder 14, **dadurch gekennzeichnet, dass** die kristallinen Salze von AHU-377 mit Aminen, die aus der Gruppe tert-Butylamin, *iso*-Propylamin, Cyclohexylamin ausgewählt sind, chemisch in das Natriumsalz der Formel 10, worin M für Natrium steht, oder in das Calciumsalz der Formel 11, worin M für Calcium steht, umgewandelt werden.

16. Verfahren zur Herstellung des amorphen Natriumsalzes von AHU-377 der Formel 10, worin M für Natrium steht, nach den Ansprüchen 13, 14 oder 15, **dadurch gekennzeichnet, dass** die kristallinen Salze von AHU-377 mit Aminen der Formel 9 mit einem Agens behandelt werden, welches aus der Gruppe anorganischer oder organischer Natriumsalze, vorzugsweise Natriumalkoholate oder Natriumhydroxid, ausgewählt ist.

17. Verfahren nach den Ansprüchen 13 bis 16, **dadurch gekennzeichnet, dass** eine Natrium-Ausgangsverbindung verwendet wird, bei der es sich um Natriummethanolat, Natriumethanolat, Natrium-iso-propoxid oder Natrium-tert-Butylat handelt.

18. Verfahren zur Herstellung des kristallinen oder halbkristallinen Calciumsalzes von AHU-377 der Formel 11, worin M für Calcium steht, nach den Ansprüchen 13 bis 17, **dadurch gekennzeichnet, dass** die kristallinen Salze von AHU-377 mit Aminen der Formel 9 mit einem Agens behandelt werden, das aus der Gruppe anorganischer oder organischer Calciumsalze, vorzugsweise Calciumalkoholate oder Calciumhydroxid, ausgewählt ist.

19. Verfahren nach den Ansprüchen 13 bis 18, **dadurch gekennzeichnet, dass** eine Calcium-Ausgangsverbindung verwendet wird, bei der es sich um Calciumchlorid, Calciumcarbonat oder Calciumacetat handelt.

## Revendications

1. Un procédé de préparation de sels cristallins de AHU-377 avec des amines de formule générale 9, dans laquelle R1, R2, R3 désignent, indépendamment les uns des autres, un groupe alkyle en C1-C6 ou un atome d'hydrogène, **caractérisé en ce que** l'acide AHU-377 décrit par la formule 1 est mis à réagir avec l'amine respective dans au moins un solvant organique ou dans un mélange le contenant, suivie de l'isolement du sel cristallin de l'amine avec AHU-377 de formule 9 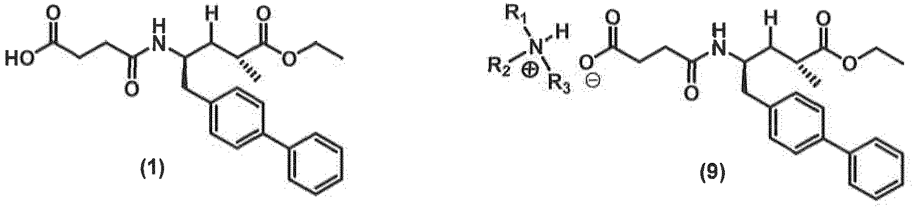 et dans laquelle l'amine utilisée est choisie dans le groupe comprenant la *tert-*butylamine, l'*iso*-propylamine, la cyclohexylamine, la *n*-propylamine et la dicyclohexylamine.

2. Le procédé selon la revendication 1, **caractérisé en ce que** la préparation des sels cristallins de AHU-377 avec des amines comprend les étapes suivantes:
(a) la réaction d'amines avec l'acide carboxylique d'AHU-377 de formule 1, dans une solution d'au moins un solvant organique, ou dans un mélange le contenant,
(b) l'isolement ultérieur des sels cristallins de formule 9, dans laquelle l'amine contenue est choisie dans le groupe comprenant la *tert*-butylamine, *l'iso-*propylamine, la cyclohexylamine, la *n*-propylamine et la dicyclohexylamine, et
(c) la recristallisation.

3. Le procédé selon la revendication 1 ou 2, **caractérisé en ce que** l'on utilise au moins un solvant organique choisi dans le groupe comprenant les esters d'acides organiques en C₁-C₅ avec des alcools en C₁-C₆, des alcools en C₁-C₆, des cétones en C₃-C₆, éthers en C₄-C₆, hydrocarbures chlorés en C₁-C₆, hydrocarbures aromatiques liquides, alcanes en C₁-C₇, cycloalcanes en C₁-C₇, ou leurs mélanges, de préférence acétate d'éthyle, acétate d'*iso*-propyle, méthanol, éthanol, alcool *iso*-propylique, acétone, méthyle éthyle cétone, l'éther diéthylique, le tétrahydrofurane, le 2-méthyltétrahydrofurane, l'éther *tert*-butylméthylique, le dichlorométhane, le toluène, les xylènes, l'hexane, l'heptane, le cyclohexane ou leurs mélanges.

4. Un procédé de recristallisation des sels cristallins de l'AHU-377 avec des amines de formule générale 9 selon les revendications 1 ou 2, **caractérisé en ce que** l'on utilise au moins un solvant organique choisi dans le groupe comprenant les esters d'acides organiques en C₁-C₅ avec des alcools en C₁₋C₆, des alcools en C₁-C₆, des cétones en C₃-C₆, des éthers en C₄-C₆, des hydrocarbures chlorés en C₁-C₆, des hydrocarbures aromatiques liquides, des alcanes en C₁-C₇, des cycloalcanes en C₁-C₇, ou leurs mélanges, de préférence l'acétate d'éthyle, l'acétate d'iso propyle, le méthanol, l'éthanol, l'alcool *iso*-propylique, l'acétone, la méthyléthylcétone, l'éther diéthylique, le tétrahydrofuranne, le 2-méthyltétrahydrofurane, l'éther *tert-*butylméthylique, le dichlorométhane, le toluène, les xylènes, l'hexane, l'heptane, le cyclohexane, ou leurs mélanges.

5. Les sels cristallins de AHU-377 avec des amines de formule 9 tel que définie selon la revendication 1, dans lesquels ce sel comprend une amine primaire choisie dans le groupe comprenant la cyclohexylamine, *l'iso*-propylamine et la *tert-*butylamine, la *n*-propylamine et la dicydohexylamine.

6. Le sel cristallin de AHU-377 selon la revendication 5 avec de la cyclohexylamine, **caractérisé par** les réflexions suivantes dans DRXP: 7,4; 15,0; 18,0; 19,9; 21,6 (° ± 0,2°2θ).

7. Le sel cristallin de AHU-377 selon la revendication 5 avec de *l'iso-*propylamine, **caractérisé par** les réflexions suivantes dans DRXP: 3,1; 9,5; 12,6; 18,5; ,24,6 (° ± 0,2°2θ).

8. Le sel cristallin de AHU-377 selon la revendication 5 avec de la *tert-*butylamine, **caractérisé par** les réflexions suivantes dans DRXP: 8,0; 9,8; 16,1; 17,8; 20,2 (° ± 0,2°2θ).

9. Un procédé de préparation et d'isolement des sels cristallins de l'AHU-377 avec les amines définies aux revendications 5, 6, 7 ou 8, **caractérisé en ce qu'**il comprend les étapes suivantes:
(a) Dissoudre l'acide AHU-377 dans un solvant organique,
(b) ajouter l'amine à la solution de l'étape (a),
(c) agiter la solution de l'étape (b),
(d) filtrer la forme solide précipitée et
(e) sécher le sel cristallin.

10. Le procédé selon la revendication 9, **caractérisé en ce que** l'on utilise au moins un solvant organique choisi dans le groupe comprenant les esters d'acides organiques en C₁ à C₅ avec des alcools en C₁ à C₆, des alcools en C₁ à C₆, des cétones en C₃ à C₆, des éthers en C₄-C₆, des hydrocarbures chlorés en C₁-C₆, des hydrocarbures aromatiques liquides, des alcanes en C₁-C₇, cycloalcanes en C₁-C₇ ou leurs mélanges, de préférence l'acétate d'éthyle, l'acétate *d'iso*-propyle, le méthanol, éthanol, l'alcool *iso*-propylique, l'acétone, la méthyl éthyle cétone, l'éther diéthylique, le tétrahydrofurane, le 2-méthyltétrahydrofurane, l'éther *tert-*butylméthylique, le dichlorométhane, le toluène, les xylènes, l'hexane, l'heptane, le cyclohexane ou leurs mélanges.

11. Le procédé selon les revendications 9 ou 10, qui comprend les étapes suivantes:
(a) dissoudre l'acide AHU-377 dans de l'acétate d'éthyle ou de l'acétate d'*iso-*propyle,
(b) ajouter à la solution de l'étape (a), 0,9 à 1,2 équivalent d'une amine choisie dans le groupe comprenant la cydohexylamine, *l'iso*-propylamine ou la *tert-*butylamine,
(c) agiter la solution de l'étape (b),
(d) filtrer la forme solide précipitée et
(e) sécher le sel cristallin sous vide.

12. Un procédé d'élimination des impuretés chimiques des sels cristallins de l'AHU-377 avec des amines obtenues selon les revendications 9, 10 ou 11, **caractérisé par** la recristallisation de sels bruts d'un solvant organique, de préférence d'acétate d'éthyle ou d'acétate *d'iso*-propyle.

13. Un procédé de préparation de formes solides pharmaceutiquement acceptables des substances de formule 10, dans laquelle M représente un métal alcalino-terreux, et 11, dans lequel M représente un métal alcalino-terreux, dérivé de la substance AHU -377 de formule 1, **caractérisé en ce que** l'on fait réagir des sels de AHU-377 avec des amines obtenues selon l'une quelconque des revendications 1 à 4 et 9 à 11 avec le sel inorganique ou organique respectif, de préférence un alcoolate ou un hydroxyde d'un métal du groupe des métaux alcalins ou des métaux alcalino-terreux

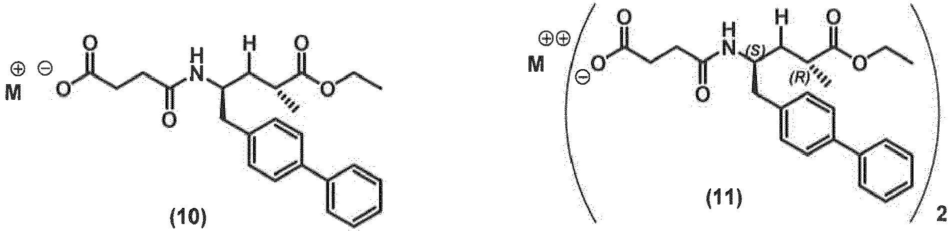

14. Le procédé selon la revendication 13, **caractérisé en ce que** la forme pharmaceutiquement acceptable est le sel de sodium de AHU-377 de formule 10, dans laquelle M représente le sodium ou le sel de calcium de AHU-377 de formule 11, dans laquelle M représente le calcium.

15. Le procédé selon les revendications 13 ou 14, **caractérisé en ce que** les sels cristallins de AHU-377 avec des amines choisies dans le groupe constitué par la *tert-*butylamine, *l'iso*-propylamine et la cyclohexylamine sont transformés chimiquement en sel de sodium de formule 10, dans laquelle M représente le sodium ou le sel de calcium de formule 11, dans laquelle M représente le calcium.

16. Le procédé de préparation du sel de sodium amorphe de AHU-377 de formule 10, dans lequel M représente le sodium, selon les revendications 13, 14 ou 15, **caractérisé en ce que** les sels cristallins de AHU-377 avec des amines de formule 9 sont traités avec un agent choisi dans le groupe des sels de sodium inorganiques ou organiques, de préférence des alcoolates de sodium ou de l'hydroxyde de sodium.

17. Le procédé selon les revendications 13 à 16, **caractérisé en ce que** l'on utilise un composé sodique source qui est le méthanolate de sodium, l'éthanolate de sodium, *l'iso*-propoxyde de sodium ou le *tert*-butylate de sodium.

18. Un procédé de préparation du sel de calcium cristallin ou semi-cristallin de AHU-377 de formule 11, dans lequel M représente le calcium, selon les revendications 13 à 17, **caractérisé en ce que** les sels cristallins de AHU -377 avec des amines de formule 9 sont traités avec un agent choisi dans le groupe des sels de calcium inorganiques ou organiques, de préférence des alcoolates de calcium et l'hydroxyde de calcium.

19. Le procédé selon les revendications 13 à 18, **caractérisé en ce que** l'on utilise un composé source de calcium qui est le chlorure de calcium, le carbonate de calcium ou l'acétate de calcium.
